(19) Europäisches Patentamt / European Patent Office / Office européen des brevets

(11) **EP 3 620 052 A1**

(12) **EUROPEAN PATENT APPLICATION**


(43) Date of publication:
**11.03.2020 Bulletin 2020/11**

(51) Int Cl.:
***A01N 43/653*** *(2006.01)* ***C07D 401/06*** *(2006.01)*
***A01P 3/00*** *(2006.01)*

(21) Application number: **18211902.4**

(22) Date of filing: **12.12.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**



(71) Applicant: **Bayer Aktiengesellschaft**
**51373 Leverkusen (DE)**

(72) Inventor: **The designation of the inventor has not yet been filed**

(74) Representative: **BIP Patents**
**c/o Bayer Intellectual Property GmbH**
**Alfred-Nobel-Straße 10**
**40789 Monheim am Rhein (DE)**


# (54) USE OF PHENOXYPYRIDINYL-SUBSTITUTED (1H-1,2,4-TRIAZOL-1-YL)ALCOHOLS FOR CONTROLLING FUNGICIDAL DISEASES IN MAIZE


(57) The present invention relates to a method of controlling fungi causing rust or leaf disease in maize by applying a phenoxypyridinyl-substituted (1H-1,2,4-triazol-1-yl)alcohol of formula (I)

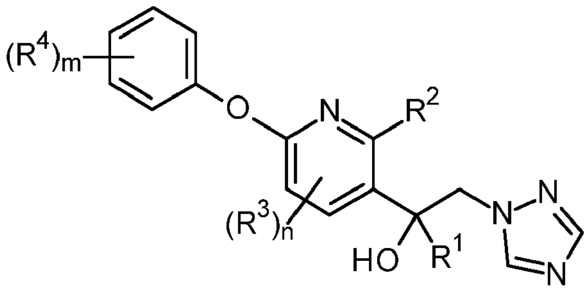

(I),

wherein $R^1$, $R^2$, $R^3$, $R^4$, m and n are defined as disclosed in the specification, to the maize plant or a part thereof via spray application.


EP 3 620 052 A1

## Description

The present invention relates to the use of specific phenoxypyridinyl-substituted (1H-1,2,4-triazol-1-yl)alcohols for controlling fungi causing rust or leaf disease in maize by spray application of said compound to the maize plant or a part thereof and to a method of controlling fungi causing rust or leaf disease in maize by spray application of said compound to the maize plant or a part thereof.

A broad range of substituted (1H-1,2,4-triazol-1-yl)alcohols are known to be useful in the field of crop protection, in particular as fungicides. WO 2017/029179 A1 discloses (1H-1,2,4-triazol-1-yl)alcohols, bearing a phenoxyheteroaryl moiety, wherein the heteroaryl ring is selected from a list of substituted or non-substituted 6-membered aromatic heterocycles containing 1 or 2 nitrogen atom(s) as heteroatom(s). WO 2017/029179 A1 further discloses processes for preparing said (1H-1,2,4-triazol-1-yl)alcohols and their use for controlling harmful microorganisms, in particular phytopathogenic harmful fungi, in crop protection and in the protection of materials. Many pathogens causing fungal diseases which can be controlled by applying said (1H-1,2,4-triazol-1-yl)alcohols are mentioned in WO 2017/029179 A1 and a long list of plants which can be treated is given. Albeit many use examples are provided in WO 2017/029179 A1, there is no specific example for the treatment of maize.

It has now been found that (1H-1,2,4-triazol-1-yl)alcohols, bearing a specifically substituted phenoxypyridinyl moiety, show high efficacy against a broad spectrum of pathogens causing rust or leaf diseases in maize, if applied via spray application. This is rather surprising, since typical maize pathogens are known to be difficult to control by well-established azole-type fungicides.

Accordingly, subject of this invention is a method of controlling fungi causing rust or leaf disease in maize plants, comprising spray application of a compound of formula (I)

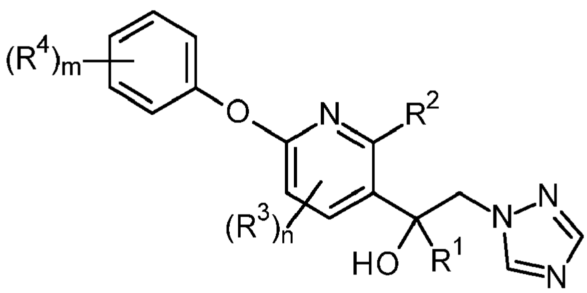

(I),

wherein

$R^1$ represents hydrogen, $C_1$-$C_6$-alkyl or $C_3$-$C_8$-cycloalkyl, wherein the $C_1$-$C_6$-alkyl may carry 1, 2, 3 or up to the maximum possible number of identical or different groups $R^a$ which independently of one another are selected from halogen, CN, nitro, phenyl, $C_1$-$C_4$-alkoxy and $C_1$-$C_4$-halogenalkoxy, and wherein the $C_3$-$C_8$-cycloalkyl may carry 1, 2, 3, 4, 5 or up to the maximum number of identical or different groups $R^b$ which independently of one another are selected from halogen, CN, nitro, $C_1$-$C_4$-alkyl, $C_1$-$C_4$-alkoxy, $C_1$-$C_4$-halogenalkyl and $C_1$-$C_4$-halogenalkoxy;

$R^2$ represents $C_1$-$C_2$-halogenalkyl or halogen;

each $R^3$ represents independently from each other halogen, CN, nitro, $C_1$-$C_4$-alkyl, $C_1$-$C_4$-halogenalkyl, $C_1$-$C_4$-alkoxy or $C_1$-$C_4$-halogenalkoxy;

n is an integer and is 0 or 1;

each $R^4$ represents independently of one another halogen, CN, nitro, $C_1$-$C_4$-alkyl, $C_1$-$C_4$-halogenalkyl, $C_1$-$C_4$-alkoxy, $C_1$-$C_4$-halogenalkoxy, $C_1$-$C_4$-alkylcarbonyl, hydroxy-substituted $C_1$-$C_4$-alkyl, pentafluoro-$\lambda^6$-sulfanyl, $C_3$-$C_6$-cycloalkyl, $C_3$-$C_6$-halogencycloalkyl or $C_1$-$C_4$-alkyl-$C_3$-$C_6$-cycloalkyl; and

m is an integer and is 0, 1 or 2;

to the maize plant or a part thereof.

In the context of the present invention, "controlling fungi" means a reduction in infestation by the respective

fungus, compared with the untreated plant measured as fungicidal efficacy, preferably a reduction by 25-50 %, compared with the untreated plant (100 %), more preferably a reduction by 40-79 %, compared with the untreated plant (100 %). Even more preferably, the infection by the fungus is suppressed by 80-100 %. Controlling comprises curative control, i.e. treatment of already infected plants in order to reduce or eliminate infection, and protective/preventive control, i.e. treatment of plants which have not yet been infected in order to prevent infection.

Surprisingly, spray application of a compound of formula (I) does not only result in effective control of pathogens causing rust or leaf diseases in maize, but also in increased plant yield.

Increasing plant yield is defined referring to total biomass per hectare, yield per hectare, in particular grain yield per hectare, kernel/fruit weight, seed size and/or hectolitre weight as well as to increased product quality, to comprise improved processability relating to size distribution (kernel, fruit, etc.), homogenous riping, grain moisture, better milling, better vinification, better brewing, increased juice yield, harvestability, digestibility, sedimentation value, falling number, pod stability, storage stability, improved fiber length/strength/uniformity, increase of milk and/or meet quality of silage fed animals, adaption to cooking and frying.

Preferably, according to this invention increasing plant yield refers to an increase in total biomass per hectare, canopy coverage and/or grain yield per hectare.

Formula (I) provides a general definition of the triazole derivatives used in the method according to the invention. Preferred radical definitions for the formulae shown above and below are given below.

$R^1$ preferably represents hydrogen, $C_1$-$C_4$-alkyl or cyclopropyl,
wherein the $C_1$-$C_4$-alkyl may carry 1, 2, 3 or up to the maximum possible number of identical or different groups $R^a$ which independently of one another are selected from halogen, CN, nitro, phenyl, $C_1$-$C_4$-alkoxy and $C_1$-$C_4$-halogenalkoxy,
and wherein the cyclopropyl may carry 1, 2, 3, 4, 5 or up to the maximum number of identical or different groups $R^b$ which independently of one another are selected from halogen, CN, nitro, $C_1$-$C_4$-alkyl, $C_1$-$C_4$-alkoxy, $C_1$-$C_4$-halogenalkyl and $C_1$-$C_4$-halogenalkoxy.

$R^1$ more preferably represents hydrogen, $C_1$-$C_4$-alkyl or cyclopropyl,
wherein the $C_1$-$C_4$-alkyl may carry 1, 2, 3 or up to the maximum possible number of identical or different groups $R^a$ which independently of one another are selected from halogen, CN, nitro, phenyl, $C_1$-$C_4$-alkoxy and $C_1$-$C_4$-halogenalkoxy,
and wherein the cyclopropyl may carry 1 or 2 identical or different groups $R^b$ which independently of one another are selected from fluorine, chlorine, bromine, CN, nitro, methyl, ethyl, n-propyl, isopropyl, n-butyl or *tert*.-butyl.

$R^1$ more preferably represents hydrogen, methyl, ethyl, n-propyl, isopropyl, n-butyl, *tert*.-butyl or cyclopropyl,
wherein the methyl, ethyl, n-propyl, isopropyl, n-butyl and *tert.-butyl* groups may carry 1, 2, 3 or up to the maximum possible number of identical or different groups $R^a$ which independently of one another are selected from fluorine, chlorine, bromine, CN or nitro,
and wherein the cyclopropyl may carry 1 or 2 identical or different groups $R^b$ which independently of one another are selected from fluorine, chlorine, bromine, CN, nitro, methyl, ethyl, n-propyl, isopropyl, n-butyl or *tert*.-butyl.

$R^1$ more preferably represents hydrogen, methyl, $CF_3$, ethyl, n-propyl, isopropyl, n-butyl, *tert.-butyl,* cyclopropyl, 1-fluorocyclopropyl, 1-chlorocyclopropyl or 1-methylcyclopropyl.

$R^1$ more preferably represents hydrogen, methyl or ethyl.

$R^1$ more preferably represents hydrogen or methyl.

$R^1$ most preferably represents methyl.

Each $R^4$ preferably represents independently from each other fluorine, chlorine, bromine, methyl, ethyl, n-propyl, iso-propyl, $CF_3$, $OCF_3$, pentafluoro-$\lambda^6$-sulfanyl, cyclopropyl, 1-halogencyclopropyl or 1-$C_1$-$C_4$-alkylcyclopropyl.

Each $R^4$ more preferably represents independently from each other Br, Cl, $CF_3$, $OCF_3$, pentafluoro-$\lambda^6$-sulfanyl, cyclopropyl, 1-fluorocyclopropyl, 1-chlorocyclopropyl or 1-methylcyclopropyl.

Each $R^4$ more preferably represents independently from each other $CF_3$, $OCF_3$, Br, Cl or pentafluoro-$\lambda^6$-sulfanyl.

Each $R^4$ most preferably represents independently from each other Br or Cl.

Preferably, $R^4$ is located in the 2- and/or 4-position of the phenyl moiety of formula (I).

More preferably, $R^4$ is located in the 4-position of the phenyl moiety of formula (I).

m preferably is 1 or 2.

m most preferably is 1.

$R^2$ preferably represents $C_1$-halogenalkyl or halogen.

$R^2$ more preferably represents Ci-halogenalkyl, F or Cl.

$R^2$ more preferably represents $CF_3$ or Cl.

$R^2$ most preferably represents $CF_3$.

Each $R^3$ preferably represents independently from each other F, Cl, Br, CN, nitro, methyl, $CF_3$, methoxy or $OCF_3$. n preferably is 0.

The radical definitions and explanations given above in general terms or stated within preferred ranges can be combined with one another as desired, i.e. including between the particular ranges and preferred ranges. They apply both to the end products and correspondingly to educts and intermediates.

Preference is given to those cases in which each of the radicals have the abovementioned preferred definitions.

Particular preference is given to those cases in which each of the radicals have the abovementioned more and/or most preferred definitions.

Hence, particular preferred the compound of formula (I) applied in the method according to the invention is selected from the group consisting of 2-[6-(4-chlorophenoxy)-2-(trifluoromethyl)-3-pyridyl]-1-(1,2,4-triazol-1-yl)propan-2-ol (1.01) and 2-[6-(4-bromophenoxy)-2-(trifluoromethyl)-3-pyridyl]-1-(1,2,4-triazol-1-yl)propan-2-ol (1.02).

Compounds of formula (I), including compounds (1.01) and (1.02) comprise a stereogenic center at the carbon atom bearing the hydroxy group. They can be present in form of the optical isomers, their racemic or scalemic mixtures (the term "scalemic" denotes a mixture of enantiomers in different proportions), in all proportions. Compounds of formula (I) may be used in the method according to this invention in any of said forms.

Particularly preferred the compound of formula (I) is selected from the group consisting of (2R)-2-[6-(4-chlorophenoxy)-2-(trifluoromethyl)-3-pyridyl]-1-(1,2,4-triazol-1-yl)propan-2-ol, (2S)-2-[6-(4-chlorophenoxy)-2-(trifluoromethyl)-3-pyridyl]-1-(1,2,4-triazol-1-yl)propan-2-ol, the racemate of (2R)-2-[6-(4-chlorophenoxy)-2-(trifluoromethyl)-3-pyridyl]-1-(1,2,4-triazol-1-yl)propan-2-ol and (2S)-2-[6-(4-chlorophenoxy)-2-(trifluoromethyl)-3-pyridyl]-1-(1,2,4-triazol-1-yl)propan-2-ol, any scalemic mixture of (2R)-2-[6-(4-chlorophenoxy)-2-(trifluoromethyl)-3-pyridyl]-1-(1,2,4-triazol-1-yl)propan-2-ol and (2S)-2-[6-(4-chlorophenoxy)-2-(trifluoromethyl)-3-pyridyl]-1-(1,2,4-triazol-1-yl)propan-2-ol, (2R)-2-[6-(4-bromophenoxy)-2-(trifluoromethyl)-3-pyridyl]-1-(1,2,4-triazol-1-yl)propan-2-ol, (2S)-2-[6-(4-bromophenoxy)-2-(trifluoromethyl)-3-pyridyl]-1-(1,2,4-triazol-1-yl)propan-2-ol, the racemate of (2R)-2-[6-(4-bromophenoxy)-2-(trifluoromethyl)-3-pyridyl]-1-(1,2,4-triazol-1-yl)propan-2-ol and (2S)-2-[6-(4-bromophenoxy)-2-(trifluoromethyl)-3-pyridyl]-1-(1,2,4-triazol-1-yl)propan-2-ol, and any scalemic mixture of (2R)-2-[6-(4-bromophenoxy)-2-(trifluoromethyl)-3-pyridyl]-1-(1,2,4-triazol-1-yl)propan-2-ol and (2S)-2-[6-(4-bromophenoxy)-2-(trifluoromethyl)-3-pyridyl]-1-(1,2,4-triazol-1-yl)propan-2-ol.

In the definitions of the symbols given in the above and below formulae, collective terms were used which are generally representative of the following substituents:
The definition $C_1$-$C_6$-alkyl comprises the largest range defined here for an alkyl radical. Specifically, this definition comprises the meanings methyl, ethyl, n-, isopropyl, n-, iso-, sec-, tert-butyl, and also in each case all isomeric pentyls and hexyls, such as methyl, ethyl, propyl, 1-methylethyl, butyl, 1-methylpropyl, 2-methylpropyl, 1,1-dimethylethyl, n-pentyl, 1-methylbutyl, 2-methylbutyl, 3-methylbutyl, 1,2-dimethylpropyl, 1,1-dimethylpropyl, 2,2-dimethylpropyl, 1-ethylpropyl, n-hexyl, 1-methylpentyl, 2-methylpentyl, 3-methylpentyl, 4-methylpentyl, 1,2-dimethylbutyl, 1,3-dimethylbutyl, 2,3-dimethylbutyl, 1,1-dimethylbutyl, 2,2-dimethylbutyl, 3,3-dimethylbutyl, 1,1,2-trimethylpropyl, 1,2,2-trimethylpropyl, 1-ethylbutyl, 2-ethylbutyl, 1-ethyl-3-methylpropyl, in particular propyl, 1-methylethyl, butyl, 1-methylbutyl, 2-methylbutyl, 3-methylbutyl, 1,1-dimethylethyl, 1,2-dimethylbutyl, 1,3-dimethylbutyl, n-pentyl, 1-methylbutyl, 1-ethylpropyl, hexyl, 3-methylpentyl. A preferred range is $C_1$-$C_4$-alkyl, such as methyl, ethyl, n-, isopropyl, n-, iso-, sec-, tert-butyl. The definition $C_1$-$C_2$-alkyl comprises methyl and ethyl.

The definition halogen comprises fluorine, chlorine, bromine and iodine.

Halogen-substituted alkyl - e.g. referred to as halogenalkyl, halogenoalkyl or haloalkyl, e.g. $C_1$-$C_4$-halogenalkyl or $C_1$-$C_2$-halogenalkyl - represents, for example, $C_1$-$C_4$-alkyl or $C_1$-$C_2$-alkyl as defined above substituted by one or more halogen substituents which can be the same or different. Preferably $C_1$-$C_4$-halogenalkyl represents chloromethyl, dichloromethyl, trichloromethyl, fluoromethyl, difluoromethyl, trifluoromethyl, chlorofluoromethyl, dichlorofluoromethyl, chlorodifluoromethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, 1,1-difluoroethyl, pentafluoroethyl, 1-fluoro-1-methylethyl,

2-fluoro-1,1-dimethylethyl, 2-fluoro-1-fluoromethyl-1-methylethyl, 2-fluoro-1,1-di(fluoromethyl)-ethyl, 1-chlorobutyl. Preferably $C_1$-$C_2$-halogenalkyl represents chloromethyl, dichloromethyl, trichloromethyl, fluoromethyl, difluoromethyl, trifluoromethyl, chlorofluoromethyl, dichlorofluoromethyl, chlorodifluoromethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, 1,1-difluoroethyl, pentafluoroethyl.

Mono- or multiple fluorinated $C_1$-$C_4$-alkyl represents, for example, $C_1$-$C_4$-alkyl as defined above substituted by one or more fluorine substituent(s). Preferably mono- or multiple fluorinated $C_1$-$C_4$-alkyl represents fluoromethyl, difluoromethyl, trifluoromethyl, 1-fluoroethyl, 2-fluoroethyl, 2,2-difluoroethyl, 2,2,2-trifluoroethyl, pentafluoroethyl, 1-fluoro-1-methylethyl, 2-fluoro-1,1-dimethylethyl, 2-fluoro-1-fluoromethyl-1-methylethyl, 2-fluoro-1,1-di(fluoromethyl)-ethyl, 1-methyl-3-trifluoromethylbutyl, 3-methyl-1-trifluoromethylbutyl.

The definition $C_2$-$C_6$-alkenyl comprises the largest range defined here for an alkenyl radical. Specifically, this definition comprises the meanings ethenyl, n-, isopropenyl, n-, iso-, sec-, tert-butenyl, and also in each case all isomeric pentenyls, hexenyls, 1-methyl-1-propenyl, 1-ethyl-1-butenyl. Halogen-substituted alkenyl - referred to as $C_2$-$C_6$-haloalkenyl - represents, for example, $C_2$-$C_6$-alkenyl as defined above substituted by one or more halogen substituents which can be the same or different.

The definition $C_2$-$C_6$-alkynyl comprises the largest range defined here for an alkynyl radical. Specifically, this definition comprises the meanings ethynyl, n-, isopropynyl, n-, iso-, sec-, tert-butynyl, and also in each case all isomeric pentynyls, hexynyls. Halogen-substituted alkynyl - referred to as $C_2$-$C_6$-haloalkynyl - represents, for example, $C_2$-$C_6$-alkynyl as defined above substituted by one or more halogen substituents which can be the same or different.

The definition $C_3$-$C_8$-cycloalkyl comprises monocyclic saturated hydrocarbyl groups having 3 to 8 carbon ring members, such as cyclopropyl, cyclobutyl, cyclopentyl, cyclohexyl, cycloheptyl and cyclooctyl.

The definition aryl comprises aromatic, mono-, bi- or tricyclic ring, for example phenyl, naphthyl, anthracenyl (anthryl), phenanthracenyl (phenanthryl).

In the method according to the invention maize plants or parts thereof are treated with a compound of formula (I).

Plant parts as understood herein are all above ground parts and organs of plants such as shoot, leaf and blossom, whereby for example leaves, stems, blossoms, fruiting bodies, fruits and seed are listed.

Generally, all maize plants, including wild plants, cultivars and plant varieties (whether or not protectable by plant variety or plant breeder's rights) can be treated. Cultivars and plant varieties can be maize plants obtained by conventional propagation and breeding methods which can be assisted or supplemented by one or more biotechnological methods such as by use of double haploids, protoplast fusion, random and directed mutagenesis, molecular or genetic markers or by bioengineering and genetic engineering methods.

Maize varieties that can be treated by the method according to the invention include for example commercially available varieties with tolerance to herbicides like "Roundup Ready Corn" and "Roundup Ready 2" from Monsanto, "Agrisure GT", "Agrisure GT/CB/LL", "Agrisure GT/RW", "Agrisure3000GT" from Syngenta, "YieldGard VT Rootworm/RR2" and "YieldGard VT Triple" from Monsanto with tolerance to glyphosate, and the corn varieties "Liberty Link" from Bayer, "Herculex I", "Herculex RW", "Herculex Xtra" from Dow, Pioneer, "Agrisure GT/CB/LL" and "Agrisure CB/LL/RW" from Syngenta with tolerance to glufosinate.

Maize varieties that can be treated by the method according to the invention further include for example commercially available varieties capable of expression of bacterial toxins like "YieldGard corn rootworm" (Monsanto), "YieldGard VT" (Monsanto), "Herculex RW" (Dow, Pioneer), "Herculex Rootworm" (Dow, Pioneer) and "Agrisure CRW" (Syngenta) with resistance against corn rootworm and the varieties "YieldGard corn borer" (Monsanto), "YieldGard VT Pro" (Monsanto), "Agrisure CB/LL" (Syngenta), "Agrisure 3000GT" (Syngenta), "Hercules I", "Hercules II" (Dow, Pioneer), "KnockOut" (Novartis), "NatureGard" (Mycogen) and "StarLink" (Aventis) with resistance against corn borer, the varieties "Herculex I" (Dow, Pioneer) and "Herculex Xtra" (Dow, Pioneer) with resistance against western bean cutworm, corn borer, black cutworm and fall armyworm, and the variety "YieldGard Plus" (Monsanto) with resistance against corn borer and corn rootworm.

Maize varieties that can be treated by the method according to the invention further include for example commercially available varieties with two combined desired properties like "YieldGard Roundup Ready" and YieldGard Roundup Ready 2" (Monsanto) with glyphosate tolerance and resistance to corn borer; the variety "Agrisure CB/LL" (Syntenta) with glufosinate tolerance and corn borer resistance; the variety "Yield Gard VT Root-worm/RR2" with glyphosate tolerance and corn rootworm resistance; the variety "Yield Gard VT Triple" with glyphosate tolerance and resistance against corn rootworm and corn borer; the variety "Herculex I" with glufosinate tolerance and lepidopteran resistance (Cry1 F), i.e. against western bean cutworm, corn borer, black cutworm and fall armyworm; the variety "YieldGard Corn Rootworm/Roundup Ready 2" (Monsanto) with glyphosate tolerance and corn rootworm resistance; the corn variety "Agrisure GT/RW" (Syngenta) with glufosinate tolerance and lepidopteran resistance (Cry3A), i.e. against western corn rootworm, northern corn rootworm and Mexican corn rootworm; the corn variety "Herculex RW" (Dow, Pioneer) with glufosinate tolerance and lepidopteran resistance (Cry34/35Ab1), i.e. against western corn rootworm, northern corn rootworm and Mexican corn rootworm; the corn variety "Yield Gard VT Rootworm/RR2" with

glyphosate tolerance and corn rootworm resistance; the corn variety "Mavera high-value corn" with glyphosate tolerance, resistance to corn rootworm and European corn borer and high lysine trait.

Maize varieties that can be treated by the method according to the invention further include for example varieties with three traits like the corn variety "Herculex I / Roundup Ready 2" with glyphosate tolerance, glufosinate tolerance and lepidopteran resistance (Cry1 F), i.e. against western bean cutworm, corn borer, black cutworm and fall armyworm; the corn variety "YieldGard Plus / Roundup Ready 2" (Monsanto) with glyphosate tolerance, corn rootworm resistance and corn borer resistance; the corn variety "Agrisure GT/CB/LL" (Syngenta) with tolerance to glyphosate, tolerance to glufosinate and corn borer resistance; the corn variety "Herculex Xtra" (Dow, Pioneer) with glufosinate tolerance and lepidopteran resistance (Cry1 F + Cry34/35Ab1), i.e. against western corn rootworm, northern corn rootworm, Mecxican corn rootworm, western bean cutworm, corn borer, black cutworm and fall armyworm; the corn varieties "Agrisure CB/LL/RW" (Syngenta) with glufosinate tolerance, corn borer resistance (Cry1Ab) and lepidopteran resistance (Cry3A), i.e. against western corn root worm, northern corn rootworm and Mexican corn rootworm; the corn variety "Agrisure 3000GT" (Syngenta) with glyphosate tolerance, corn borer resistance (Cry1Ab) and lepidopteran resistance (Cry3A), i.e. against western corn rootworm, northern corn rootworm and Mexican corn rootworm.

Maize varieties that can be treated by the method according to the invention further include for example varieties with four traits like "Hercules Quad-Stack" (Dow, Pioneer) with glyphosate tolerance, glufosinate tolerance, corn borer resistance and corn rootworm resistance.

Preferably, in the method according to the invention the maize plant is selected from the Pioneer maize varieties P30F53, P30F53 RR - Roundup Ready®, P9608AM LL RR2 CRW ECB and P30F53YHV, the Dekalb® maize varieties DKB 390, DKB 390 IPRO3, DKC 35-88 RIB RR2 BT and DKC 62-08, the variety P7958 AM RR2 LL BT available from Marc Hutlet Seeds Ltd., the variety NK1120-3122 RR+GLU+ECB+CRW available from Syngenta and the variety Golden Bantam.

The method according to the invention allows control of fungi causing rust or leaf diseases in maize.

Preferably, the fungus to be controlled is selected from fungi causing rust, leaf blotch, leaf wilt, leaf spot or blight diseases.

More preferably, the fungus is selected from *Cercospora* species, *Phaeosphaeria* species, *Helminthosporium* species, *Puccinia* species, *Cochliobolus* species, *Kabatiella* species and *Setosphaeria* species.

Most preferably, the fungus is selected from *Cercospora zeae maydis, Phaeosphaeria maydis, Helminthosporium spp., Puccinia sorghi, Puccinia polysora, Cochliobolus heterostrophus, Kabatiella zeae* and *Setosphaeria turcica.*

In the method according to the invention the compound of formula (I) is applied via spray application. This allows very uniform application of the compound of formula (I) to the plants even in case of dense plant coverage.

In order to be applicable via spray application the compound of formula (I) needs to be provided in sprayable form. Preferably, the compound of formula (I) is provided as composition / formulation, comprising the compound of formula (I) and at least one suitable liquid carrier.

Suitable liquid carriers are preferably selected from water, organic solvents and combinations thereof. More preferred, the liquid carrier is water or a mixture of water and an organic solvent.

Examples of suitable organic solvents include polar and nonpolar organic chemical liquids, for example from the classes of aromatic and nonaromatic hydrocarbons (such as cyclohexane, paraffins, alkylbenzenes, xylene, toluene alkylnaphthalenes, chlorinated aromatics or chlorinated aliphatic hydrocarbons such as chlorobenzenes, chloroethylenes or methylene chloride), alcohols and polyols , which may optionally also be substituted, etherified and/or esterified, (such as butanol or glycol), ketones (such as acetone, methyl ethyl ketone, methyl isobutyl ketone or cyclohexanone), esters (including fats and oils) and (poly)ethers, unsubstituted and substituted amines, amides (such as dimethylformamide), lactams (such as N-alkylpyrrolidones) and lactones, sulfones and sulfoxides (such as dimethyl sulfoxide).

The amount of liquid carrier typically ranges from 1 to 99.99%, preferably from 5 to 99.9%, more preferably from 10 to 99.5%, and most preferably from 20 to 99% by weight of the composition.

Preferably, the composition further comprises at least one surfactant.

The surfactant can be an ionic (cationic or anionic) or non-ionic surfactant, such as ionic or non-ionic emulsifier(s), foam former(s), dispersant(s), wetting agent(s) and any mixtures thereof. Examples of suitable surfactants include, but are not limited to, salts of polyacrylic acid, salts of lignosulfonic acid, salts of phenolsulfonic acid or naphthalenesulfonic acid, polycondensates of ethylene and/or propylene oxide with fatty alcohols, fatty acids or fatty amines (polyoxyethylene fatty acid esters, polyoxyethylene fatty alcohol ethers, for example alkylaryl polyglycol ethers), substituted phenols (preferably alkylphenols or arylphenols), salts of sulfosuccinic esters, taurine derivatives (preferably alkyl taurates), phosphoric esters of polyethoxylated alcohols or phenols, fatty esters of polyols and derivatives of compounds containing sulfates, sulfonates, phosphates (for example, alkylsulfonates, alkyl sulfates, arylsulfonates) and protein hydrolysates, lignosulfite waste liquors and methylcellulose. The amount of surfactants typically ranges from 0.5 to 40, preferably 5 to 40% by weight of the composition.

The composition may also comprise at least one further auxiliary.

Examples of suitable auxiliaries include water repellents, siccatives, binders (adhesive, tackifier, fixing agent,

such as carboxymethylcellulose, natural and synthetic polymers in the form of powders, granules or latices, such as gum arabic, polyvinyl alcohol and polyvinyl acetate, natural phospholipids such as cephalins and lecithins and synthetic phospholipids, polyvinylpyrrolidone and tylose), thickeners, stabilizers (e.g. cold stabilizers, preservatives, antioxidants, light stabilizers, or other agents which improve chemical and/or physical stability), dyes or pigments (such as inorganic pigments, e.g. iron oxide, titanium oxide and Prussian Blue; organic dyes, e.g. alizarin, azo and metal phthalocyanine dyes), antifoams (e.g. silicone antifoams and magnesium stearate), preservatives (e.g. dichlorophene and benzyl alcohol hemiformal), secondary thickeners (such as cellulose derivatives, acrylic acid derivatives, xanthan, modified clays and finely divided silica), stickers, gibberellins and processing auxiliaries, mineral and vegetable oils, perfumes, waxes, nutrients (including trace nutrients, such as salts of iron, manganese, boron, copper, cobalt, molybdenum and zinc), protective colloids, thixotropic substances, penetrants, sequestering agents and complex formers.

The composition may be in any customary sprayable form, such as solutions (e.g aqueous solutions), emulsions, water- and oil-based suspensions, suspoemulsions and the respective concentrates. The compound of formula (I) may be present in a suspended, emulsified or dissolved form.

The composition may be provided to the end user as ready-for-use formulation, i.e. a composition that may be directly applied to the plants or plant parts by a suitable spraying device. Alternatively, the compositions may be provided to the end user in the form of concentrates which have to be diluted, preferably with water, prior to use.

The composition useful in the method according to the invention contains generally from 0.01 to 99% by weight, preferably from 0.02 to 98% by weight, more preferably from 0.05 to 95% by weight, more preferably from 0.1 to 90% by weight, most preferably from 0.1 to 80% by weight of the compound of formula (I). It is possible that a composition comprises two or more compounds of formula (I). In such case the outlined ranges refer to the total amount of compounds of formula (I).

Preferably, the compound of formula (I) is provided as an emulsifiable concentrate or suspension concentrate, more preferably an emulsifiable concentrate comprising the compound of formula (I) in an amount of 20 to 400 g/L, preferably 40 to 200 g/L, or a suspension concentrate comprising the compound of formula (I) in an amount of 50 to 500 g/L, preferably 100 to 400 g/L.

Preparation of said concentrates is well known to those skilled in the art. For example, emulsifiable concentrates (EC) can be prepared by dissolving the desired amount of compound of formula (I), e.g. 20 to 400 g per litre concentrate, and 50 to 100 g per litre concentrate of at least one surfactant in a water-insoluble organic solvent, e.g. an aromatic hydrocarbon, or a water-soluble organic solvent, e.g. N-methyl-2-pyrrolidone (NMP), dimethylsulfoxide (DMSO) and γ-butyrolactone. Suspension concentrates (SC) can be prepared by mixing the desired amount of compound of formula (I), e.g. 50 to 500 g per litre concentrate, 20 to 100 g per litre concentrate of at least one surfactant and 1 to 20 g per litre concentrate of at least one binder and/or secondary thickener and suspending this mixture in water.

Preferably, before applying said concentrates to the maize plant or a part thereof, the concentrate is diluted with water. More preferably, the emulsifiable concentrate or suspension concentrate is mixed with water in such amount to arrive at a concentration of the compound of formula (I) in the resulting mixture of 0.1 to 5, preferably 0.2 to 2, more preferred 0.25 to 1 g/l.

Preferably, the compound of formula (I) is applied to the maize plant or part thereof in an amount corresponding to 50 to 300 g, preferably 50 to 150 g, more preferably 60 to 125 g compound of formula (I) per hectare treated area.

In the method according to the invention, the compound of formula (I) is preferably applied to the plant or part thereof 1 to 10 times, more preferably 1 to 8 times, more preferably 1 to 5 times, most preferably 1 to 2 times.

The interval between 2 treatments is preferably from 1 day to 4 weeks, more preferred from 5 days to 3 weeks, most preferred from 10 to 20 days.

Preferably, the compound of formula (I) is applied in the growth stages BBCH 10 to 89, more preferred in growth stages BBCH 30 to 70. More preferred, at least one application is done during growth stages 50 to 70.

The term "growth stage" refers to the growth stages as defined by the BBCH Codes in "Growth stages of mono- and dicotyledonous plants", 2nd edition 2001, edited by Uwe Meier from the Federal Biological Research Centre for Agriculture and Forestry. The BBCH codes are a well-established system for a uniform coding of similar growth stages of all mono- and dicotyledonous plant species. The abbreviation BBCH derives from "Biologische Bundesanstalt, Bundessortenamt und Chemische Industrie".

In the method according to the invention the compound of formula (I) may be applied together with other active substances, e. g. with fungicides, bactericides, acaricides, nematicides, insecticides, herbicides, fertilizers, growth regulators, safeners or semiochemicals, for example as pre-mix or tank mix. Especially suitable mixtures that can be used according to the present invention and mixing partners or further active ingredients that may be preferably present together with any one of the compounds of formula (I) are detailed in WO 2018/145921 A1.

Examples of especially preferred fungicides which can be mixed with the compound and the composition used in the method of the invention are:

1) Inhibitors of the ergosterol biosynthesis, for example (1.001) cyproconazole, (1.002) difenoconazole, (1.003)

epoxiconazole, (1.004) fenhexamid, (1.005) fenpropidin, (1.006) fenpropimorph, (1.007) fenpyrazamine, (1.008) fluquinconazole, (1.009) flutriafol, (1.010) imazalil, (1.011) imazalil sulfate, (1.012) ipconazole, (1.013) metconazole, (1.014) myclobutanil, (1.015) paclobutrazol, (1.016) prochloraz, (1.017) propiconazole, (1.018) prothioconazole, (1.019) Pyrisoxazole, (1.020) spiroxamine, (1.021) tebuconazole, (1.022) tetraconazole, (1.023) triadimenol, (1.024) tridemorph, (1.025) triticonazole, (1.026) (1R,2S,5S)-5-(4-chlorobenzyl)-2-(chloromethyl)-2-methyl-1-(1H-1,2,4-triazol-1-ylmethyl)cyclopentanol, (1.027) (1S,2R,5R)-5-(4-chlorobenzyl)-2-(chloromethyl)-2-methyl-1-(1H-1,2,4-triazol-1-ylmethyl)cyclopentanol, (1.028) (2R)-2-(1-chlorocyclopropyl)-4-[(1R)-2,2-dichlorocyclopropyl]-1-(1H-1,2,4-triazol-1-yl)butan-2-ol, (1.029) (2R)-2-(1-chlorocyclopropyl)-4-[(1S)-2,2-dichlorocyclopropyl]-1-(1H-1,2,4-triazol-1-yl)butan-2-ol, (1.030) (2R)-2-[4-(4-chlorophenoxy)-2-(trifluoromethyl)phenyl]-1-(1H-1,2,4-triazol-1-yl)propan-2-ol, (1.031) (2S)-2-(1-chlorocyclopropyl)-4-[(1R)-2,2-dichlorocyclopropyl]-1-(1H-1,2,4-triazol-1-yl)butan-2-ol, (1.032) (2S)-2-(1-chlorocyclopropyl)-4-[(1S)-2,2-dichlorocyclopropyl]-1-(1H-1,2,4-triazol-1-yl)butan-2-ol, (1.033) (2S)-2-[4-(4-chlorophenoxy)-2-(trifluoromethyl)phenyl]-1-(1H-1,2,4-triazol-1-yl)propan-2-ol, (1.034) (R)-[3-(4-chloro-2-fluorophenyl)-5-(2,4-difluorophenyl)-1,2-oxazol-4-yl](pyridin-3-yl)methanol, (1.035) (S)-[3-(4-chloro-2-fluorophenyl)-5-(2,4-difluorophenyl)-1,2-oxazol-4-yl](pyridin-3-yl)methanol, (1.036) [3-(4-chloro-2-fluorophenyl)-5-(2,4-difluorophenyl)-1,2-oxazol-4-yl] (pyridin-3-yl)methanol, (1.037) 1-({(2R,4S)-2- [2-chloro-4-(4-chlorophenoxy)phenyl] -4-methyl-1,3-dioxolan-2-yl}methyl)-1H-1,2,4-triazole, (1.038) 1-({(2S,4S)-2-[2-chloro-4-(4-chlorophenoxy)phenyl]-4-methyl-1,3-dioxolan-2-yl}methyl)-1H-1,2,4-triazole, (1.039) 1-{[3-(2-chlorophenyl)-2-(2,4-difluorophenyl)oxiran-2-yl]methyl}-1H-1,2,4-triazol-5-yl thiocyanate, (1.040) 1-{[rel(2R,3R)-3-(2-chlorophenyl)-2-(2,4-difluorophenyl)oxiran-2-yl]methyl}-1H-1,2,4-triazol-5-yl thiocyanate, (1.041) 1-{[rel(2R,3S)-3-(2-chlorophenyl)-2-(2,4-difluorophenyl)oxiran-2-yl]methyl} -1H-1,2,4-triazol-5-yl thiocyanate, (1.042) 2-[(2R,4R,5R)-1-(2,4-dichlorophenyl)-5-hydroxy-2,6,6-trimethylheptan-4-yl]-2,4-dihydro-3H-1,2,4-triazole-3-thione, (1.043) 2-[(2R,4R,5S)-1-(2,4-dichlorophenyl)-5-hydroxy-2,6,6-trimethylheptan-4-yl]-2,4-dihydro-3H-1,2,4-triazole-3-thione, (1.044) 2-[(2R,4S,5R)-1-(2,4-dichlorophenyl)-5-hydroxy-2,6,6-trimethylheptan-4-yl]-2,4-dihydro-3H-1,2,4-triazole-3-thione, (1.045) 2-[(2R,4S,5S)-1-(2,4-dichlorophenyl)-5-hydroxy-2,6,6-trimethylheptan-4-yl]-2,4-dihydro-3H-1,2,4-triazole-3-thione, (1.046) 2-[(2S,4R,5R)-1-(2,4-dichlorophenyl)-5-hydroxy-2,6,6-trimethylheptan-4-yl]-2,4-dihydro-3H-1,2,4-triazole-3-thione, (1.047) 2-[(2S,4R,5S)-1-(2,4-dichlorophenyl)-5-hydroxy-2,6,6-trimethylheptan-4-yl]-2,4-dihydro-3H-1,2,4-triazole-3-thione, (1.048) 2-[(2S,4S,5R)-1-(2,4-dichlorophenyl)-5-hydroxy-2,6,6-trimethylheptan-4-yl]-2,4-dihydro-3H-1,2,4-triazole-3-thione, (1.049) 2-[(2S,4S,5S)-1-(2,4-dichlorophenyl)-5-hydroxy-2,6,6-trimethylheptan-4-yl]-2,4-dihydro-3H-1,2,4-triazole-3-thione, (1.050) 2-[1-(2,4-dichlorophenyl)-5-hydroxy-2,6,6-trimethylheptan-4-yl]-2,4-dihydro-3H-1,2,4-triazole-3-thione, (1.051) 2-[2-chloro-4-(2,4-dichlorophenoxy)phenyl]-1-(1H-1,2,4-triazol-1-yl)propan-2-ol, (1.052) 2-[2-chloro-4-(4-chlorophenoxy)phenyl]-1-(1H-1,2,4-triazol-1-yl)butan-2-ol, (1.053) 2-[4-(4-chlorophenoxy)-2-(trifluoromethyl)phenyl]-1-(1H-1,2,4-triazol-1-yl)butan-2-ol, (1.054) 2-[4-(4-chlorophenoxy)-2-(trifluoromethyl)phenyl]-1-(1H-1,2,4-triazol-1-yl)pentan-2-ol, (1.055) Mefentrifluconazole, (1.056) 2-{[3-(2-chlorophenyl)-2-(2,4-difluorophenyl)oxiran-2-yl]methyl}-2,4-dihydro-3H-1,2,4-triazole-3-thione, (1.057) 2-{[rel(2R,3R)-3-(2-chlorophenyl)-2-(2,4-difluorophenyl)oxiran-2-yl]methyl}-2,4-dihydro-3H-1,2,4-triazole-3-thione, (1.058) 2-{ [rel(2R,3S)-3-(2-chlorophenyl)-2-(2,4-difluorophenyl)oxiran-2-yl]methyl}-2,4-dihydro-3H-1,2,4-triazole-3-thione, (1.059) 5-(4-chlorobenzyl)-2-(chloromethyl)-2-methyl-1-(1H-1,2,4-triazol-1-ylmethyl)cyclopentanol, (1.060) 5-(allylsulfanyl)-1-{[3-(2-chlorophenyl)-2-(2,4-difluorophenyl)oxiran-2-yl]methyl}-1H-1,2,4-triazole, (1.061) 5-(allylsulfanyl)-1-{[rel(2R,3R)-3-(2-chlorophenyl)-2-(2,4-difluorophenyl)oxiran-2-yl]methyl}-1H-1,2,4-triazole, (1.062) 5-(allylsulfanyl)-1-{[rel(2R,3S)-3-(2-chlorophenyl)-2-(2,4-difluorophenyl)oxiran-2-yl]methyl}-1H-1,2,4-triazole, (1.063) N'-(2,5-dimethyl-4-{ [3-(1,1,2,2-tetrafluoroethoxy)phenyl]sulfanyl}phenyl)-N-ethyl-N-methylimidoformamide, (1.064) N'-(2,5-dimethyl-4-{[3-(2,2,2-trifluoroethoxy)phenyl]sulfanyl}phenyl)-N-ethyl-N-methylimidoformamide, (1.065) N'-(2,5-dimethyl-4-{[3-(2,2,3,3-tetrafluoropropoxy)phenyl]sulfanyl}phenyl)-N-ethyl-N-methylimidoformamide, (1.066) N'-(2,5-dimethyl-4-{[3-(pentafluoroethoxy)phenyl]sulfanyl}phenyl)-N-ethyl-N-methylimidoformamide, (1.067) N'-(2,5-dimethyl-4-{3-[(1,1,2,2-tetrafluoroethyl)sulfanyl]phenoxy}phenyl)-N-ethyl-N-methylimidoformamide, (1.068) N'-(2,5-dimethyl-4-{3-[(2,2,2-trifluoroethyl)sulfanyl]phenoxy}phenyl)-N-ethyl-N-methylimidoformamide, (1.069) N'-(2,5-dimethyl-4-{3-[(2,2,3,3-tetrafluoropropyl)sulfanyl]phenoxy}phenyl)-N-ethyl-N-methylimidoformamide, (1.070) N'-(2,5-dimethyl-4-{3-[(pentafluoroethyl)sulfanyl]phenoxy}phenyl)-N-ethyl-N-methylimidoformamide, (1.071) N'-(2,5-dimethyl-4-phenoxyphenyl)-N-ethyl-N-methylimidoformamide, (1.072) N'-(4-{ [3-(difluoromethoxy)phenyl] sulfanyl} -2,5-dimethylphenyl)-N-ethyl-N-methylimidoformamide, (1.073) N'-(4-{3-[(difluoromethyl)sulfanyl]phenoxy}-2,5-dimethylphenyl)-N-ethyl-N-methylimidoformamide, (1.074) N'-[5-bromo-6-(2,3-dihydro-1H-inden-2-yloxy)-2-methylpyridin-3-yl]-N-ethyl-N-methylimidoformamide, (1.075) N'-{4-[(4,5-dichloro-1,3-thiazol-2-yl)oxy]-2,5-dimethylphenyl} -N-ethyl-N-methylimidoformamide, (1.076) N'-{5-bromo-6-[(1R)-1-(3,5-difluorophenyl)ethoxy]-2-methylpyridin-3-yl}-N-ethyl-N-methylimidoformamide, (1.077) N'-{5-bromo-6-[(1S)-1-(3,5-difluorophenyl)ethoxyl-2-methylpyridin-3-yl}-N-ethyl-N-methylimidoformamide, (1.078) N'-{5-bromo-6-[(cis-4-isopropylcyclohexyl)oxy]-2-methylpyridin-3-yl}-N-ethyl-N-methylimidoformamide, (1.079) N'-{5-bromo-6-[(trans-4-isopropylcyclohexyl)oxy]-2-methylpyridin-3-yl}-N-ethyl-N-methylimidoformamide, (1.080) N'-{5-bromo-6-[1-(3,5-difluorophenyl)ethoxy]-2-methylpyridin-3-yl}-N-ethyl-N-methylimidoformamide, (1.081) Ipfentrifluconazole.

2) Inhibitors of the respiratory chain at complex I or II, for example (2.001) benzovindiflupyr, (2.002) bixafen, (2.003) boscalid, (2.004) carboxin, (2.005) fluopyram, (2.006) flutolanil, (2.007) fluxapyroxad, (2.008) furametpyr, (2.009) Isofetamid, (2.010) isopyrazam (anti-epimeric enantiomer 1R,4S,9S), (2.011) isopyrazam (anti-epimeric enantiomer 1S,4R,9R), (2.012) isopyrazam (anti-epimeric racemate 1RS,4SR,9SR), (2.013) isopyrazam (mixture of syn-epimeric racemate 1RS,4SR,9RS and anti-epimeric racemate 1RS,4SR,9SR), (2.014) isopyrazam (syn-epimeric enantiomer 1R,4S,9R), (2.015) isopyrazam (syn-epimeric enantiomer 1S,4R,9S), (2.016) isopyrazam (syn-epimeric racemate 1RS,4SR,9RS), (2.017) penflufen, (2.018) penthiopyrad, (2.019) pydiflumetofen, (2.020) Pyraziflumid, (2.021) sedaxane, (2.022) 1,3-dimethyl-N-(1,1,3-trimethyl-2,3-dihydro-1H-inden-4-yl)-1H-pyrazole-4-carboxamide, (2.023) 1,3-dimethyl-N-[(3R)-1,1,3-trimethyl-2,3-dihydro-1H-inden-4-yl]-1H-pyrazole-4-carboxamide, (2.024) 1,3-dimethyl-N-[(3S)-1,1,3-trimethyl-2,3-dihydro-1H-inden-4-yl]-1H-pyrazole-4-carboxamide, (2.025) 1-methyl-3-(trifluoromethyl)-N-[2'-(trifluoromethyl)biphenyl-2-yl]-1H-pyrazole-4-carboxamide, (2.026) 2-fluoro-6-(trifluoromethyl)-N-(1,1,3-trimethyl-2,3-dihydro-1H-inden-4-yl)benzamide, (2.027) 3-(difluoromethyl)-1-methyl-N-(1,1,3-trimethyl-2,3-dihydro-1H-inden-4-yl)-1H-pyrazole-4-carboxamide, (2.028) 3-(difluoromethyl)-1-methyl-N-[(3R)-1,1,3-trimethyl-2,3-dihydro-1H-inden-4-yl]-1H-pyrazole-4-carboxamide, (2.029) 3-(difluoromethyl)-1-methyl-N-[(3S)-1,1,3-trimethyl-2,3-dihydro-1H-inden-4-yl]-1H-pyrazole-4-carboxamide, (2.030) Fluindapyr, (2.031) 3-(difluoromethyl)-N-[(3R)-7-fluoro-1,1,3-trimethyl-2,3-dihydro-1H-inden-4-yl]-1-methyl-1H-pyrazole-4-carboxamide, (2.032) 3-(difluoromethyl)-N-[(3S)-7-fluoro-1,1,3-trimethyl-2,3-dihydro-1H-inden-4-yl]-1-methyl-1H-pyrazole-4-carboxamide, (2.033) 5,8-difluoro-N-[2-(2-fluoro-4-{ [4-(trifluoromethyl)pyridin-2-yl]oxy}phenyl)ethyl]quinazolin-4-amine, (2.034) N-(2-cyclopentyl-5-fluorobenzyl)-N-cyclopropyl-3-(difluoromethyl)-5-fluoro-1-methyl-1H-pyrazole-4-carboxamide, (2.035) N-(2-tert-butyl-5-methylbenzyl)-N-cyclopropyl-3-(difluoromethyl)-5-fluoro-1-methyl-1H-pyrazole-4-carboxamide, (2.036) N-(2-tert-butylbenzyl)-N-cyclopropyl-3-(difluoromethyl)-5-fluoro-1-methyl-1H-pyrazole-4-carboxamide, (2.037) N-(5-chloro-2-ethylbenzyl)-N-cyclopropyl-3-(difluoromethyl)-5-fluoro-1-methyl-1H-pyrazole-4-carboxamide, (2.038) N-(5-chloro-2-isopropylbenzyl)-N-cyclopropyl-3-(difluoromethyl)-5-fluoro-1-methyl-1H-pyrazole-4-carboxamide, (2.039) N-[(1R,4S)-9-(dichloromethylene)-1,2,3,4-tetrahydro-1,4-methanonaphthalen-5-yl] -3-(difluoromethyl)-1-methyl-1H-pyrazole-4-carboxamide, (2.040) N-[(1S,4R)-9-(dichloromethylene)-1,2,3,4-tetrahydro-1,4-methanonaphthalen-5-yl]-3-(difluoromethyl)-1-methyl-1H-pyrazole-4-carboxamide, (2.041) N-[1-(2,4-dichlorophenyl)-1-methoxypropan-2-yl]-3-(difluoromethyl)-1-methyl-1H-pyrazole-4-carboxamide, (2.042) N-[2-chloro-6-(trifluoromethyl)benzyl]-N-cyclopropyl-3-(difluoromethyl)-5-fluoro-1-methyl-1H-pyrazole-4-carboxamide, (2.043) N-[3-chloro-2-fluoro-6-(trifluoromethyl)benzyl]-N-cyclopropyl-3-(difluoromethyl)-5-fluoro-1-methyl-1H-pyrazole-4-carboxamide, (2.044) N-[5-chloro-2-(trifluoromethyl)benzyl]-N-cyclopropyl-3-(difluoromethyl)-5-fluoro-1-methyl-1H-pyrazole-4-carboxamide, (2.045) N-cyclopropyl-3-(difluoromethyl)-5-fluoro-1-methyl-N-[5-methyl-2-(trifluoromethyl)benzyl]-1H-pyrazole-4-carboxamide, (2.046) N-cyclopropyl-3-(difluoromethyl)-5-fluoro-N-(2-fluoro-6-isopropylbenzyl)-1-methyl-1H-pyrazole-4-carboxamide, (2.047) N-cyclopropyl-3-(difluoromethyl)-5-fluoro-N-(2-isopropyl-5-methylbenzyl)-1-methyl-1H-pyrazole-4-carboxamide, (2.048) N-cyclopropyl-3-(difluoromethyl)-5-fluoro-N-(2-isopropylbenzyl)-1-methyl-1H-pyrazole-4-carbothioamide, (2.049) N-cyclopropyl-3-(difluoromethyl)-5-fluoro-N-(2-isopropylbenzyl)-1-methyl-1H-pyrazole-4-carboxamide, (2.050) N-cyclopropyl-3-(difluoromethyl)-5-fluoro-N-(5-fluoro-2-isopropylbenzyl)-1-methyl-1H-pyrazole-4-carboxamide, (2.051) N-cyclopropyl-3-(difluoromethyl)-N-(2-ethyl-4,5-dimethylbenzyl)-5-fluoro-1-methyl-1H-pyrazole-4-carboxamide, (2.052) N-cyclopropyl-3-(difluoromethyl)-N-(2-ethyl-5-fluorobenzyl)-5-fluoro-1-methyl-1H-pyrazole-4-carboxamide, (2.053) N-cyclopropyl-3-(difluoromethyl)-N-(2-ethyl-5-methylbenzyl)-5-fluoro-1-methyl-1H-pyrazole-4-carboxamide, (2.054) N-cyclopropyl-N-(2-cyclopropyl-5-fluorobenzyl)-3-(difluoromethyl)-5-fluoro-1-methyl-1H-pyrazole-4-carboxamide, (2.055) N-cyclopropyl-N-(2-cyclopropyl-5-methylbenzyl)-3-(difluoromethyl)-5-fluoro-1-methyl-1H-pyrazole-4-carboxamide, (2.056) N-cyclopropyl-N-(2-cyclopropylbenzyl)-3-(difluoromethyl)-5-fluoro-1-methyl-1H-pyrazole-4-carboxamide, (2.057) pyrapropoyne.

3) Inhibitors of the respiratory chain at complex III, for example (3.001) ametoctradin, (3.002) amisulbrom, (3.003) azoxystrobin, (3.004) coumethoxystrobin, (3.005) coumoxystrobin, (3.006) cyazofamid, (3.007) dimoxystrobin, (3.008) enoxastrobin, (3.009) famoxadone, (3.010) fenamidone, (3.011) flufenoxystrobin, (3.012) fluoxastrobin, (3.013) kresoxim-methyl, (3.014) metominostrobin, (3.015) orysastrobin, (3.016) picoxystrobin, (3.017) pyraclostrobin, (3.018) pyrametostrobin, (3.019) pyraoxystrobin, (3.020) trifloxystrobin, (3.021) (2E)-2-{2-[({[(1E)-1-(3-{[(E)-1-fluoro-2-phenylvinyl]oxy}phenyl)ethylidene]amino}oxy)methyl]phenyl}-2-(methoxyimino)-N-methylacetamide, (3.022) (2E,3Z)-5-{[1-(4-chlorophenyl)-1H-pyrazol-3-yl]oxy}-2-(methoxyimino)-N,3-dimethylpent-3-enamide, (3.023) (2R)-2- {2- [(2,5-dimethylphenoxy)methyl]phenyl} -2-methoxy-N-methylacetamide, (3.024) (2S)-2-{2-[(2,5-dimethylphenoxy)methyl]phenyl}-2-methoxy-N-methylacetamide, (3.025) (3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoate, (3.026) mandestrobin, (3.027) N-(3-ethyl-3,5,5-trimethylcyclohexyl)-3-formamido-2-hydroxybenzamide, (3.028) (2E,3Z)-5-{[1-(4-chloro-2-fluorophenyl)-1H-pyrazol-3-yl]oxy}-2-(methoxyimino)-N,3-dimethylpent-3-enamide, (3.029) methyl {5-[3-(2,4-dimethylphenyl)-1H-pyrazol-1-yl]-2-methylbenzyl}carbamate, (3.030) metyltetraprole.

4) Inhibitors of the mitosis and cell division, for example (4.001) carbendazim, (4.002) diethofencarb, (4.003) ethaboxam, (4.004) fluopicolide, (4.005) pencycuron, (4.006) thiabendazole, (4.007) thiophanate-methyl, (4.008) zoxamide, (4.009) 3-chloro-4-(2,6-difluorophenyl)-6-methyl-5-phenylpyridazine, (4.010) 3-chloro-5-(4-chlorophenyl)-4-(2,6-difluorophenyl)-6-methylpyridazine, (4.011) 3-chloro-5-(6-chloropyridin-3-yl)-6-methyl-4-(2,4,6-trifluorophenyl)pyridazine, (4.012) 4-(2-bromo-4-fluorophenyl)-N-(2,6-difluorophenyl)-1,3-dimethyl-1H-pyrazol-5-amine, (4.013) 4-(2-bromo-4-fluorophenyl)-N-(2-bromo-6-fluorophenyl)-1,3-dimethyl-1H-pyrazol-5-amine, (4.014) 4-(2-bromo-4-fluorophenyl)-N-(2-bromophenyl)-1,3-dimethyl-1H-pyrazol-5-amine, (4.015) 4-(2-bromo-4-fluorophenyl)-N-(2-chloro-6-fluorophenyl)-1,3-dimethyl-1H-pyrazol-5-amine, (4.016) 4-(2-bromo-4-fluorophenyl)-N-(2-chlorophenyl)-1,3-dimethyl-1H-pyrazol-5-amine, (4.017) 4-(2-bromo-4-fluorophenyl)-N-(2-fluorophenyl)-1,3-dimethyl-1H-pyrazol-5-amine, (4.018) 4-(2-chloro-4-fluorophenyl)-N-(2,6-difluorophenyl)-1,3-dimethyl-1H-pyrazol-5-amine, (4.019) 4-(2-chloro-4-fluorophenyl)-N-(2-chloro-6-fluorophenyl)-1,3-dimethyl-1H-pyrazol-5-amine, (4.020) 4-(2-chloro-4-fluorophenyl)-N-(2-chlorophenyl)-1,3-dimethyl-1H-pyrazol-5-amine, (4.021) 4-(2-chloro-4-fluorophenyl)-N-(2-fluorophenyl)-1,3-dimethyl-1H-pyrazol-5-amine, (4.022) 4-(4-chlorophenyl)-5-(2,6-difluorophenyl)-3,6-dimethylpyridazine, (4.023) N-(2-bromo-6-fluorophenyl)-4-(2-chloro-4-fluorophenyl)-1,3-dimethyl-1H-pyrazol-5-amine, (4.024) N-(2-bromophenyl)-4-(2-chloro-4-fluorophenyl)-1,3-dimethyl-1H-pyrazol-5-amine, (4.025) N-(4-chloro-2,6-difluorophenyl)-4-(2-chloro-4-fluorophenyl)-1,3-dimethyl-1H-pyrazol-5-amine.

5) Compounds capable to have a multisite action, for example (5.001) bordeaux mixture, (5.002) captafol, (5.003) captan, (5.004) chlorothalonil, (5.005) copper hydroxide, (5.006) copper naphthenate, (5.007) copper oxide, (5.008) copper oxychloride, (5.009) copper(2+) sulfate, (5.010) dithianon, (5.011) dodine, (5.012) folpet, (5.013) mancozeb, (5.014) maneb, (5.015) metiram, (5.016) metiram zinc, (5.017) oxine-copper, (5.018) propineb, (5.019) sulfur and sulfur preparations including calcium polysulfide, (5.020) thiram, (5.021) zineb, (5.022) ziram, (5.023) 6-ethyl-5,7-dioxo-6,7-dihydro-5H-pyrrolo[3',4':5,6][1,4]dithiino[2,3-c][1,2]thiazole-3-carbonitrile.

6) Compounds capable to induce a host defence, for example (6.001) acibenzolar-S-methyl, (6.002) isotianil, (6.003) probenazole, (6.004) tiadinil.

7) Inhibitors of the amino acid and/or protein biosynthesis, for example (7.001) cyprodinil, (7.002) kasugamycin, (7.003) kasugamycin hydrochloride hydrate, (7.004) oxytetracycline, (7.005) pyrimethanil, (7.006) 3-(5-fluoro-3,3,4,4-tetramethyl-3,4-dihydroisoquinolin-1-yl)quinoline.

8) Inhibitors of the ATP production, for example (8.001) silthiofam.

9) Inhibitors of the cell wall synthesis, for example (9.001) benthiavalicarb, (9.002) dimethomorph, (9.003) flumorph, (9.004) iprovalicarb, (9.005) mandipropamid, (9.006) pyrimorph, (9.007) valifenalate, (9.008) (2E)-3-(4-tert-butylphenyl)-3-(2-chloropyridin-4-yl)-1-(morpholin-4-yl)prop-2-en-1-one, (9.009) (2Z)-3-(4-tert-butylphenyl)-3-(2-chloropyridin-4-yl)-1-(morpholin-4-yl)prop-2-en-1-one.

10) Inhibitors of the lipid and membrane synthesis, for example (10.001) propamocarb, (10.002) propamocarb hydrochloride, (10.003) tolclofos-methyl.

11) Inhibitors of the melanin biosynthesis, for example (11.001) tricyclazole, (11.002) 2,2,2-trifluoroethyl {3-methyl-1-[(4-methylbenzoyl)amino]butan-2-yl}carbamate.

12) Inhibitors of the nucleic acid synthesis, for example (12.001) benalaxyl, (12.002) benalaxyl-M (kiralaxyl), (12.003) metalaxyl, (12.004) metalaxyl-M (mefenoxam).

13) Inhibitors of the signal transduction, for example (13.001) fludioxonil, (13.002) iprodione, (13.003) procymidone, (13.004) proquinazid, (13.005) quinoxyfen, (13.006) vinclozolin.

14) Compounds capable to act as an uncoupler, for example (14.001) fluazinam, (14.002) meptyldinocap.

15) Further compounds, for example (15.001) Abscisic acid, (15.002) benthiazole, (15.003) bethoxazin, (15.004) capsimycin, (15.005) carvone, (15.006) chinomethionat, (15.007) cufraneb, (15.008) cyflufenamid, (15.009) cymoxanil, (15.010) cyprosulfamide, (15.011) flutianil, (15.012) fosetyl-aluminium, (15.013) fosetyl-calcium, (15.014) fosetyl-sodium, (15.015) methyl isothiocyanate, (15.016) metrafenone, (15.017) mildiomycin, (15.018) natamycin, (15.019) nickel dimethyldithiocarbamate, (15.020) nitrothal-isopropyl, (15.021) oxamocarb, (15.022) oxathiapiprolin, (15.023) oxyfenthiin, (15.024) pentachlorophenol and salts, (15.025) phosphorous acid and its salts, (15.026)

propamocarb-fosetylate, (15.027) pyriofenone (chlazafenone), (15.028) tebufloquin, (15.029) tecloftalam, (15.030) tolnifanide, (15.031) 1-(4-{4-[(5R)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, (15.032) 1-(4-{4-[(5S)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, (15.033) 2-(6-benzylpyridin-2-yl)quinazoline, (15.034) dipymetitrone, (15.035) 2-[3,5-bis(difluoromethyl)-1H-pyrazol-1-yl]-1-[4-(4-{5-[2-(prop-2-yn-1-yloxy)phenyl]-4,5-dihydro-1,2-oxazol-3-yl}-1,3-thiazol-2-yl)piperidin-1-yl]ethanone, (15.036) 2-[3,5-bis(difluoromethyl)-1H-pyrazol-1-yl]-1-[4-(4-{5-[2-chloro-6-(prop-2-yn-1-yloxy)phenyl]-4,5-dihydro-1,2-oxazol-3-yl}-1,3-thiazol-2-yl)piperidin-1-yl]ethanone, (15.037) 2-[3,5-bis(difluoromethyl)-1H-pyrazol-1-yl]-1-[4-(4-{5-[2-fluoro-6-(prop-2-yn-1-yloxy)phenyl]-4,5-dihydro-1,2-oxazol-3-yl}-1,3-thiazol-2-yl)piperidin-1-yl]ethanone, (15.038) 2-[6-(3-fluoro-4-methoxyphenyl)-5-methylpyridin-2-yl]quinazoline, (15.039) 2-{(5R)-3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl methanesulfonate, (15.040) 2-{(5S)-3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl methanesulfonate, (15.041) Ipflufenoquin, (15.042) 2-{2-fluoro-6-[(8-fluoro-2-methylquinolin-3-yl)oxy]phenyl}propan-2-ol, (15.043) 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl methanesulfonate, (15.044) 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}phenyl methanesulfonate, (15.045) 2-phenylphenol and salts, (15.046) 3-(4,4,5-trifluoro-3,3-dimethyl-3,4-dihydroisoquinolin-1-yl)quinoline, (15.047) quinofumelin, (15.048) 4-amino-5-fluoropyrimidin-2-ol (tautomeric form: 4-amino-5-fluoropyrimidin-2(1H)-one), (15.049) 4-oxo-4-[(2-phenylethyl)amino]butanoic acid, (15.050) 5-amino-1,3,4-thiadiazole-2-thiol, (15.051) 5-chloro-N'-phenyl-N'-(prop-2-yn-1-yl)thiophene-2-sulfonohydrazide, (15.052) 5-fluoro-2-[(4-fluorobenzyl)oxy]pyrimidin-4-amine, (15.053) 5-fluoro-2-[(4-methylbenzyl)oxy]pyrimidin-4-amine, (15.054) 9-fluoro-2,2-dimethyl-5-(quinolin-3-yl)-2,3-dihydro-1,4-benzoxazepine, (15.055) but-3-yn-1-yl {6-[({[(Z)-(1-methyl-1H-tetrazol-5-yl)(phenyl)methylene]amino}oxy)methyl]pyridin-2-yl}carbamate, (15.056) ethyl (2Z)-3-amino-2-cyano-3-phenylacrylate, (15.057) phenazine-1-carboxylic acid, (15.058) propyl 3,4,5-trihydroxybenzoate, (15.059) quinolin-8-ol, (15.060) quinolin-8-ol sulfate (2:1), (15.061) tert-butyl{6-[({[(1-methyl-1H-tetrazol-5-yl)(phenyl)methylene]amino}oxy)methyl]pyridin-2-yl}carbamate, (15.062) 5-fluoro-4-imino-3-methyl-1-[(4-methylphenyl)sulfonyl]-3,4-dihydropyrimidin-2(1H)-one, (15.063) aminopyrifen.

All named mixing partners of the classes (1) to (15) as described here above can be present in the form of the free compound and/or, if their functional groups enable this, an agriculturally acceptable salt thereof.

In the method according to the invention the compound of formula (I) (in the following designated as component A) and the further active compound(s) selected from groups (1) to (15) (in the following designated as component B), can be present in a broad range of effective weight ratio of A:B, for example in a range of 100:1 to 1:100, preferably in a weight ratio of 50:1 to 1:50, most preferably in a weight ratio of 20:1 to 1:20. Further ratios of A:B which can be used according to the present invention with increasing preference in the order given are: 95:1 to 1:95, 90:1 to 1:90, 85:1 to 1:85, 80:1 to 1:80, 75:1 to 1:75, 70:1 to 1:70, 65:1 to 1:65, 60:1 to 1:60, 55:1 to 1:55, 45:1 to 1:45, 40:1 to 1:40, 35:1 to 1:35, 30:1 to 1:30, 25:1 to 1:25, 15:1 to 1:15, 10:1 to 1:10, 5:1 to 1:5, 4:1 to 1:4, 3:1 to 1:3, 2:1 to 1:2. In case, two or more components B are present said ratios are based on the total weight of further active compound(s) selected from groups (1) to (15) being present.

The compound and the composition used in the method of the invention may also be combined with one or more biological control agents.

Examples of biological control agents which may be present together with the compound of formula (I) in the method of the invention are:

<u>(A) Antibacterial agents selected from the group of:</u>

(A1) bacteria, such as (A1.1) *Bacillus subtilis,* in particular strain QST713/AQ713 (available as SERENADE OPTI or SERENADE ASO from Bayer CropScience LP, US, having NRRL Accession No. B21661and described in U.S. Patent No. 6,060,051); (A1.2) *Bacillus amyloliquefaciens,* in particular strain D747 (available as Double Nickel™ from Certis, US, having accession number FERM BP-8234 and disclosed in US Patent No. 7,094,592); (A1.3) *Bacillus pumilus,* in particular strain BU F-33 (having NRRL Accession No. 50185); (A1.4) *Bacillus subtilis var. amyloliquefaciens* strain FZB24 (available as Taegro® from Novozymes, US); (A1.5) a *Paenibacillus* sp. strain having Accession No. NRRL B-50972 or Accession No. NRRL B-67129 and described in International Patent Publication No. WO 2016/154297; and

(A2) fungi, such as (A2.1) *Aureobasidium pullulans,* in particular blastospores of strain DSM14940; (A2.2) *Aureo-*

*basidium pullulans* blastospores of strain DSM 14941; (A2.3) *Aureobasidium pullulans,* in particular mixtures of blastospores of strains DSM14940 and DSM14941;

(B) Fungicides selected from the group of:

(B1) bacteria, for example (B1.1) *Bacillus subtilis,* in particular strain QST713/AQ713 (available as SERENADE OPTI or SERENADE ASO from Bayer CropScience LP, US, having NRRL Accession No. B21661and described in U.S. Patent No. 6,060,051); (B1.2) *Bacillus pumilus,* in particular strain QST2808 (available as SONATA® from Bayer CropScience LP, US, having Accession No. NRRL B-30087 and described in U.S. Patent No. 6,245,551); (B1.3) *Bacillus pumilus,* in particular strain GB34 (available as Yield Shield® from Bayer AG, DE); (B1.4) *Bacillus pumilus,* in particular strain BU F-33 (having NRRL Accession No. 50185); (B1.5) *Bacillus amyloliquefaciens,* in particular strain D747 (available as Double Nickel™ from Certis, US, having accession number FERM BP-8234 and disclosed in US Patent No. 7,094,592); (B1.6) *Bacillus subtilis* Y1336 (available as BIOBAC® WP from Bion-Tech, Taiwan, registered as a biological fungicide in Taiwan under Registration Nos. 4764, 5454, 5096 and 5277); (B1.7) *Bacillus amyloliquefaciens* strain MBI 600 (available as SUBTILEX from BASF SE); (B1.8) *Bacillus subtilis* strain GB03 (available as Kodiak® from Bayer AG, DE); (B1.9) *Bacillus subtilis var. amyloliquefaciens* strain FZB24 (available from Novozymes Biologicals Inc., Salem, Virginia or Syngenta Crop Protection, LLC, Greensboro, North Carolina as the fungicide TAEGRO® or TAEGRO® ECO (EPA Registration No. 70127-5); (B1.10) *Bacillus mycoides,* isolate J (available as BmJ TGAI or WG from Certis USA); (B1.11) *Bacillus licheniformis,* in particular strain SB3086 (available as EcoGuard TM Biofungicide and Green Releaf from Novozymes); (B1.12) a *Paenibacillus* sp. strain having Accession No. NRRL B-50972 or Accession No. NRRL B-67129 and described in International Patent Publication No. WO 2016/154297.

In some embodiments, the biological control agent is a *Bacillus subtilis* or *Bacillus amyloliquefaciens* strain that produces a fengycin or plipastatin-type compound, an iturin-type compound, and/or a surfactin-type compound. For background, see the following review article: Ongena, M., et al., "Bacillus Lipopeptides: Versatile Weapons for Plant Disease Biocontrol," Trends in Microbiology, Vol 16, No. 3, March 2008, pp. 115-125. *Bacillus* strains capable of producing lipopeptides include *Bacillus subtilis* QST713 (available as SERENADE OPTI or SERENADE ASO from Bayer CropScience LP, US, having NRRL Accession No. B21661and described in U.S. Patent No. 6,060,051), *Bacillus amyloliquefaciens* strain D747 (available as Double Nickel™ from Certis, US, having accession number FERM BP-8234 and disclosed in US Patent No. 7,094,592); *Bacillus subtilis* MBI600 (available as SUBTILEX® from Becker Underwood, US EPA Reg. No. 71840-8**);** *Bacillus subtilis* Y1336 (available as BIOBAC® WP from Bion-Tech, Taiwan, registered as a biological fungicide in Taiwan under Registration Nos. 4764, 5454, 5096 and 5277); *Bacillus amyloliquefaciens,* in particular strain FZB42 (available as RHIZOVITAL® from ABiTEP, DE); and *Bacillus subtilis* var. *amyloliquefaciens* FZB24 (available from Novozymes Biologicals Inc., Salem, Virginia or Syngenta Crop Protection, LLC, Greensboro, North Carolina as the fungicide TAEGRO® or TAEGRO® ECO (EPA Registration No. 70127-5); and

(B2) fungi, for example: (B2.1) *Coniothyrium minitans,* in particular strain CON/M/91-8 (Accession No. DSM-9660; e.g. Contans ® from Bayer); (B2.2) *Metschnikowia fructicola,* in particular strain NRRL Y-30752 (e.g. Shemer®); (B2.3) *Microsphaeropsis ochracea* (e.g. Microx® from Prophyta); (B2.5) *Trichoderma spp.,* including *Trichoderma atroviride,* strain SC1 described in International Application No. PCT/IT2008/000196); (B2.6) *Trichoderma harzianum rifai* strain KRL-AG2 (also known as strain T-22, /ATCC 208479, e.g. PLANTSHIELD T-22G, Rootshield®, and TurfShield from BioWorks, US); (B2.14) *Gliocladium roseum,* strain 321U from W.F. Stoneman Company LLC; (B2.35) *Talaromyces flavus,* strain V117b; (B2.36) *Trichoderma asperellum,* strain ICC 012 from Isagro; (B2.37) *Trichoderma asperellum,* strain SKT-1 (e.g. ECO-HOPE® from Kumiai Chemical Industry); (B2.38) *Trichoderma atroviride,* strain CNCM 1-1237 (e.g. Esquive® WP from Agrauxine, FR); (B2.39) *Trichoderma atroviride,* strain no. V08/002387; (B2.40) *Trichoderma atroviride,* strain NMI no. V08/002388; (B2.41) *Trichoderma atroviride,* strain NMI no. V08/002389; (B2.42) *Trichoderma atroviride,* strain NMI no. V08/002390; (B2.43) *Trichoderma atroviride,* strain LC52 (e.g. Tenet by Agrimm Technologies Limited); (B2.44) *Trichoderma atroviride,* strain ATCC 20476 (IMI 206040); (B2.45) *Trichoderma atroviride,* strain T11 (IMI352941/ CECT20498); (B2.46) *Trichoderma harmatum;* (B2.47) *Trichoderma harzianum;* (B2.48) *Trichoderma harzianum rifai T39* (e.g. Trichodex® from Makhteshim, US); (B2.49) *Trichoderma harzianum,* in particular, strain KD (e.g. Trichoplus from Biological Control Products, SA (acquired by Becker Underwood)); (B2.50) *Trichoderma harzianum,* strain ITEM 908 (e.g. Trianum-P from Koppert); (B2.51) *Trichoderma harzianum,* strain TH35 (e.g. Root-Pro by Mycontrol); (B2.52) *Trichoderma virens* (also known as *Gliocladium virens*), in particular strain GL-21 (e.g. SoilGard 12G by Certis, US); (B2.53) *Trichoderma viride,* strain TV1(e.g. Trianum-P by Koppert); (B2.54) *Ampelomyces quisqualis,* in particular strain AQ 10 (e.g. AQ 10® by IntrachemBio Italia); (B2.56) *Aureobasidium pullulans,* in particular blastospores of strain DSM14940; (B2.57) *Aureobasidium pullulans,* in particular blastospores of strain DSM 14941; (B2.58) *Aureobasidium pullulans,* in particular

mixtures of blastospores of strains DSM14940 and DSM 14941 (e.g. Botector® by bio-ferm, CH); (B2.64) *Cladosporium cladosporioides,* strain H39 (by Stichting Dienst Landbouwkundig Onderzoek); (B2.69) *Gliocladium catenulatum* (Synonym: *Clonostachys rosea f. catenulate*) strain J1446 (e.g. Prestop ® by AgBio Inc. and also e.g. Primastop® by Kemira Agro Oy); (B2.70) *Lecanicillium lecanii* (formerly known as *Verticillium lecanii*) *conidia* of strain KV01 (e.g. Vertalec® by Koppert/Arysta); (B2.71) *Penicillium vermiculatum;* (B2.72) *Pichia anomala,* strain WRL-076 (NRRL Y-30842); (B2.75) *Trichoderma atroviride,* strain SKT-1 (FERM P-16510); (B2.76) *Trichoderma atroviride,* strain SKT-2 (FERM P-16511); (B2.77) *Trichoderma atroviride,* strain SKT-3 (FERM P-17021); (B2.78) *Trichoderma gamsii* (formerly *T. viride*), strain ICC080 (IMI CC 392151 CABI, e.g. BioDerma by AGROBIOSOL DE MEXICO, S.A. DE C.V.); (B2.79) *Trichoderma harzianum,* strain DB 103 (e.g., T-Gro 7456 by Dagutat Biolab); (B2.80) *Trichoderma polysporum,* strain IMI 206039 (e.g. Binab TF WP by BINAB Bio-Innovation AB, Sweden); (B2.81) *Trichoderma stromaticum* (e.g. Tricovab by Ceplac, Brazil); (B2.83) *Ulocladium oudemansii,* in particular strain HRU3 (e.g. Botry-Zen® by Botry-Zen Ltd, NZ); (B2.84) *Verticillium albo-atrum* (formerly *V. dahliae),* strain WCS850 (CBS 276.92; e.g. Dutch Trig by Tree Care Innovations); (B2.86) *Verticillium chlamydosporium;* (B2.87) mixtures of *Trichoderma asperellum* strain ICC 012 and *Trichoderma gamsii* strain ICC 080 (product known as e.g. BIO-TAM™ from Bayer CropScience LP, US).

Further examples of biological control agents which may be present in the method of the invention are:

bacteria selected from the group consisting of *Bacillus cereus,* in particular *B. cereus* strain CNCM 1-1562 and *Bacillus firmus,* strain 1-1582 (Accession number CNCM 1-1582), *Bacillus subtilis strain* OST 30002 (Accession No. NRRL B-50421), *Bacillus thuringiensis,* in particular *B. thuringiensis* subspecies *israelensis* (serotype H-14), strain AM65-52 (Accession No. ATCC 1276), *B. thuringiensis subsp. aizawai,* in particular strain ABTS-1857 (SD-1372), *B. thuringiensis subsp. kurstaki* strain HD-1, *B. thuringiensis subsp. tenebrionis* strain NB 176 (SD-5428), *Pasteuria penetrans, Pasteuria spp.* (Rotylenchulus reniformis nematode)-PR3 (Accession Number ATCC SD-5834), *Streptomyces microflavus* strain AQ6121 (= QRD 31.013, NRRL B-50550), and *Streptomyces galbus* strain AQ 6047 (Acession Number NRRL 30232);

fungi and yeasts selected from the group consisting of *Beauveria bassiana,* in particular strain ATCC 74040, *Lecanicillium spp.,* in particular strain HRO LEC 12, *Metarhizium anisopliae,* in particular strain F52 (DSM3884 or ATCC 90448), *Paecilomyces fumosoroseus* (now: *Isaria fumosorosea*), in particular strain IFPC 200613, or strain Apopka 97 (Accesion No. ATCC 20874), and *Paecilomyces lilacinus,* in particular *P. lilacinus* strain 251 (AGAL 89/030550);

viruses selected from the group consisting of *Adoxophyes orana* (summer fruit tortrix) granulosis virus (GV), *Cydia pomonella* (codling moth) granulosis virus (GV), *Helicoverpa armigera* (cotton bollworm) nuclear polyhedrosis virus (NPV), *Spodoptera exigua* (beet armyworm) mNPV, *Spodoptera frugiperda* (fall armyworm) mNPV, and *Spodoptera littoralis* (African cotton leafworm) NPV.

bacteria and fungi which can be added as 'inoculant' to plants or plant parts or plant organs and which, by virtue of their particular properties, promote plant growth and plant health. Examples are: *Agrobacterium spp., Azorhizobium caulinodans, Azospirillum spp., Azotobacter spp., Bradyrhizobium spp., Burkholderia spp.,* in particular *Burkholderia cepacia* (formerly known as *Pseudomonas cepacia*), *Gigaspora spp.,* or *Gigaspora monosporum, Glomus spp., Laccaria spp., Lactobacillus buchneri, Paraglomus spp., Pisolithus tinctorus, Pseudomonas spp., Rhizobium spp.,* in particular *Rhizobium trifolii, Rhizopogon spp., Scleroderma spp., Suillus spp.,* and *Streptomyces spp.*

plant extracts and products formed by microorganisms including proteins and secondary metabolites which can be used as biological control agents, such as *Allium sativum, Artemisia absinthium,* azadirachtin, Biokeeper WP, *Cassia nigricans, Celastrus angulatus, Chenopodium anthelminticum,* chitin, Armour-Zen, *Dryopteris filix-mas, Equisetum arvense,* Fortune Aza, Fungastop, Heads Up (*Chenopodium quinoa* saponin extract), *Pyrethrum*/*Pyrethrins, Quassia amara, Quercus, Quillaja,* Regalia, "Requiem ™ Insecticide", rotenone, *ryania*/ryanodine, *Symphytum officinale, Tanacetum vulgare,* thymol, Triact 70, TriCon, *Tropaeulum majus, Urtica dioica,* Veratrin, *Viscum album, Brassicaceae* extract, in particular oilseed rape powder or mustard powder.

Examples of insecticides, acaricides and nematicides, respectively, which can be present in the method of the invention, are:

(1) Acetylcholinesterase (AChE) inhibitors, such as, for example, carbamates, for example alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, triazamate, trimethacarb, XMC and xylylcarb; or organophosphates, for example acephate, azamethiphos, azinphos-ethyl, azinphos-methyl, cadusafos, chlorethoxyfos, chlorfenvinphos, chlormephos, chlorpyrifos-methyl, coumaphos, cyanophos, demeton-S-methyl, diazinon, dichlorvos/DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl O-(methoxyaminothiophosphoryl) salicylate, isoxathion, malathion, mecarbam, methamidophos, methidathion, mevinphos, monocrotophos, naled, omethoate, oxydemeton-methyl, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, triclorfon and vamidothion.

(2) GABA-gated chloride channel blockers, such as, for example, cyclodiene-organochlorines, for example chlordane and endosulfan or phenylpyrazoles (fiproles), for example ethiprole and fipronil.

(3) Sodium channel modulators, such as, for example, pyrethroids, e.g. acrinathrin, allethrin, d-cis-trans allethrin, d-trans allethrin, bifenthrin, bioallethrin, bioallethrin s-cyclopentenyl isomer, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin [(1R)-trans-isomer], deltamethrin, empenthrin [(EZ)-(1R)-isomer], esfenvalerate, etofenprox, fenpropathrin, fenvalerate, flucythrinate, flumethrin, tau-fluvalinate, halfenprox, imiprothrin, kadethrin, momfluorothrin, permethrin, phenothrin [(1R)-trans-isomer], prallethrin, pyrethrins (pyrethrum), resmethrin, silafluofen, tefluthrin, tetramethrin, tetramethrin [(1R)- isomer)], tralomethrin and transfluthrin or DDT or methoxychlor.

(4) Nicotinic acetylcholine receptor (nAChR) competitive modulators, such as, for example, neonicotinoids, e.g. acetamiprid, clothianidin, dinotefuran, imidacloprid, nitenpyram, thiacloprid and thiamethoxam or nicotine or sulfoxaflor or flupyradifurone.

(5) Nicotinic acetylcholine receptor (nAChR) allosteric modulators, such as, for example, spinosyns, e.g. spinetoram and spinosad.

(6) Glutamate-gated chloride channel (GluCl) allosteric modulators, such as, for example, avermectins/milbemycins, for example abamectin, emamectin benzoate, lepimectin and milbemectin.

(7) Juvenile hormone mimics, such as, for example, juvenile hormone analogues, e.g. hydroprene, kinoprene and methoprene or fenoxycarb or pyriproxyfen.

(8) Miscellaneous non-specific (multi-site) inhibitors, such as, for example, alkyl halides, e.g. methyl bromide and other alkyl halides; or chloropicrine or sulphuryl fluoride or borax or tartar emetic or methyl isocyanate generators, e.g. diazomet and metam.

(9) Modulators of Chordotonal Organs, such as, for example pymetrozine or flonicamid.

(10) Mite growth inhibitors, such as, for example clofentezine, hexythiazox and diflovidazin or etoxazole.

(11) Microbial disruptors of the insect gut membrane, such as, for example *Bacillus thuringiensis* subspecies *israelensis, Bacillus sphaericus, Bacillus thuringiensis* subspecies *aizawai, Bacillus thuringiensis* subspecies *kurstaki, Bacillus thuringiensis* subspecies *tenebrionis,* and *B.t.* plant proteins: Cry1Ab, Cry1Ac, Cry1Fa, Cry1A.105, Cry2Ab, Vip3A, mCry3A, Cry3Ab, Cry3Bb, Cry34Ab1/35Ab1.

(12) Inhibitors of mitochondrial ATP synthase, such as, ATP disruptors such as, for example, diafenthiuron or organotin compounds, for example azocyclotin, cyhexatin and fenbutatin oxide or propargite or tetradifon.

(13) Uncouplers of oxidative phosphorylation via disruption of the proton gradient, such as, for example, chlorfenapyr, DNOC and sulfluramid.

(14) Nicotinic acetylcholine receptor channel blockers, such as, for example, bensultap, cartap hydrochloride, thiocylam, and thiosultap-sodium.

(15) Inhibitors of chitin biosynthesis, type 0, such as, for example, bistrifluron, chlorfluazuron, diflubenzuron, flucycloxuron, flufenoxuron, hexaflumuron, lufenuron, novaluron, noviflumuron, teflubenzuron and triflumuron.

(16) Inhibitors of chitin biosynthesis, type 1, for example buprofezin.

(17) Moulting disruptor (in particular for Diptera, i.e. dipterans), such as, for example, cyromazine.

(18) Ecdysone receptor agonists, such as, for example, chromafenozide, halofenozide, methoxyfenozide and tebufenozide.

(19) Octopamine receptor agonists, such as, for example, amitraz.

(20) Mitochondrial complex III electron transport inhibitors, such as, for example, hydramethylnone or acequinocyl or fluacrypyrim.

(21) Mitochondrial complex I electron transport inhibitors, such as, for example from the group of the METI acaricides, e.g. fenazaquin, fenpyroximate, pyrimidifen, pyridaben, tebufenpyrad and tolfenpyrad or rotenone (Derris).

(22) Voltage-dependent sodium channel blockers, such as, for example indoxacarb or metaflumizone.

(23) Inhibitors of acetyl CoA carboxylase, such as, for example, tetronic and tetramic acid derivatives, e.g. spirodiclofen, spiromesifen and spirotetramat.

(24) Mitochondrial complex IV electron transport inhibitors, such as, for example, phosphines, e.g. aluminium phosphide, calcium phosphide, phosphine and zinc phosphide or cyanides, e.g. calcium cyanide, potassium cyanide and sodium cyanide.

(25) Mitochondrial complex II electron transport inhibitors, such as, for example, *beta*-ketonitrile derivatives, e.g. cyenopyrafen and cyflumetofen and carboxanilides, such as, for example, pyflubumide.

(28) Ryanodine receptor modulators, such as, for example, diamides, e.g. chlorantraniliprole, cyantraniliprole and flubendiamide,
further active compounds such as, for example, Afidopyropen, Afoxolaner, Azadirachtin, Benclothiaz, Benzoximate, Bifenazate, Broflanilide, Bromopropylate, Chinomethionat, Chloroprallethrin, Cryolite, Cyclaniliprole, Cycloxaprid, Cyhalodiamide, Dicloromezotiaz, Dicofol, epsilon-Metofluthrin, epsilon-Momfluthrin, Flometoquin, Fluazaindolizine, Fluensulfone, Flufenerim, Flufenoxystrobin, Flufiprole, Fluhexafon, Fluralaner, Fluxametamide, Fufenozide, Guadipyr, Heptafluthrin, Imidaclothiz, Iprodione, kappa-Bifenthrin, kappa-Tefluthrin, Lotilaner, Meperfluthrin, Paichongding, Pyridalyl, Pyrifluquinazon, Pyriminostrobin, Spirobudiclofen, Tetramethylfluthrin, Tetraniliprole, Tetrachlorantraniliprole, Tigolaner, Tioxazafen, Thiofluoximate, Triflumezopyrim and iodomethane; furthermore preparations based on *Bacillus firmus* (I-1582, BioNeem, Votivo), and also the following compounds: 1-{2-fluoro-4-methyl-5-[(2,2,2-trifluoroethyl)sulphinyl]phenyl}-3-(trifluoromethyl)-1H-1,2,4-triazole-5-amine (known from WO2006/043635) (CAS 885026-50-6), {1'-[(2E)-3-(4-chlorophenyl)prop-2-en-1-yl]-5-fluorospiro[indol-3,4'-piperidin]-1(2H)-yl}(2-chloropyridin-4-yl)methanone (known from WO2003/106457) (CAS 637360-23-7), 2-chloro-N-[2-{1-[(2E)-3-(4-chlorophenyl)prop-2-en-1-yl]piperidin-4-yl}-4-(trifluoromethyl)phenyl]isonicotinamide (known from WO2006/003494) (CAS 872999-66-1), 3-(4-chloro-2,6-dimethylphenyl)-4-hydroxy-8-methoxy-1,8-diazaspiro[4.5]dec-3-en-2-one (known from WO 2010052161) (CAS 1225292-17-0), 3-(4-chloro-2,6-dimethylphenyl)-8-methoxy-2-oxo-1,8-diazaspiro[4.5]dec-3-en-4-yl ethyl carbonate (known from EP2647626) (CAS 1440516-42-6), 4-(but-2-yn-1-yloxy)-6-(3,5-dimethylpiperidin-1-yl)-5-fluoropyrimidine (known from WO2004/099160) (CAS 792914-58-0), PF1364 (known from JP2010/018586) (CAS 1204776-60-2), N-[(2E)-1-[(6-chloropyridin-3-yl)methyl]pyridin-2(1H)-ylidene]-2,2,2-trifluoroacetamide (known from WO2012/029672) (CAS 1363400-41-2), (3*E*)-3-[1-[(6-chloro-3-pyridyl)methyl] -2-pyridylidene] -1,1,1-trifluoro-propan-2-one (known from WO2013/144213) (CAS 1461743-15-6),, *N*-[3-(benzylcarbamoyl)-4-chlorophenyl]-1-methyl-3-(pentafluoroethyl)-4-(trifluoromethyl)-1*H*-pyrazole-5-carboxamide (known from WO2010/051926) (CAS 1226889-14-0), 5-bromo-4-chloro-*N*-[4-chloro-2-methyl-6-(methylcarbamoyl)phenyl]-2-(3-chloro-2-pyridyl)pyrazole-3-carboxamide (known from CN103232431) (CAS 1449220-44-3), 4-[5-(3,5-dichlorophenyl)-4,5-dihydro-5-(trifluoromethyl)-3-isoxazolyl]-2-methyl-*N*-(*cis*-1-oxido-3-thietanyl)-benzamide, 4-[5-(3,5-dichlorophenyl)-4,5-dihydro-5-(trifluoromethyl)-3-isoxazolyl]-2-methyl-*N*-(*trans*-1-oxido-3-thietanyl)-benzamide and 4-[(5*S*)-5-(3,5-dichlorophenyl)-4,5-dihydro-5-(trifluoromethyl)-3-isoxazolyl]-2-methyl-*N*-(*cis*-1-oxido-3-thietanyl)benzamide (known from WO 2013/050317 A1) (CAS 1332628-83-7), *N*-[3-chloro-1-(3-pyridinyl)-1*H*-pyrazol-4-yl]-*N*-ethyl-3-[(3, 3,3-trifluoropropyl)sulfinyl]-propanamide, (+)-*N*-[3-chloro-1-(3-pyridinyl)-1*H*-pyrazol-4-yl]-*N*-ethyl-3-[(3,3,3-trifluoropropyl)sulfinyl]-propanamide and (-)-*N*-[3-chloro-1-(3-pyridinyl)-1*H*-pyrazol-4-yl]-*N*-ethyl-3-[(3,3,3-trifluoropropyl)sulfinyl]-propanamide (known from WO 2013/162715 A2, WO 2013/162716 A2, US 2014/0213448 A1) (CAS 1477923-37-7), 5-[[(2*E*)-3-chloro-2-propen-1-yl] amino]-1-[2,6-dichloro-4-(trifluoromethyl)phenyl]-4-[(trifluoromethyl)sulfinyl]-1*H*-pyrazole-3-carbonitrile (known from CN 101337937 A) (CAS 1105672-77-2), 3-bromo-*N*-[4-chloro-2-methyl-6-[(methylamino)thioxomethyl]phenyl]-1-(3-chloro-2-pyridinyl)-1*H*-pyrazole-5-carboxamide, (Liudaibenjiaxuanan, known from CN 103109816 A) (CAS 1232543-85-9); *N*-[4-chloro-2-[[(1,1-dimethylethyl)amino]carbonyl]-6-methylphenyl]-1-(3-chloro-2-pyridinyl)-3-(fluoromethoxy)-1*H*-Pyrazole-5-carboxamide (known from WO 2012/034403 A1) (CAS 1268277-22-0), *N*-[2-(5-amino-1,3,4-thiadiazol-2-yl)-4-chloro-6-methylphenyl]-3-bromo-1-(3-chloro-2-pyridinyl)-1*H*-pyrazole-5-carboxamide (known from WO 2011/085575 A1) (CAS 1233882-22-8), 4-[3-[2,6-dichloro-4-[(3,3-dichloro-2-propen-1-yl)oxy]phenoxy]propoxy]-2-methoxy-6-(trifluoromethyl)-pyrimidine (known from CN 101337940 A) (CAS 1108184-52-6); (2E)- and 2(Z)-2-[2-(4-cyanophenyl)-1-[3-(trifluoromethyl)phenyl]ethylidene]-*N*-[4-(difluoromethoxy)phenyl]-hydrazinecarboxamide (known from CN 101715774 A) (CAS 1232543-85-9); 3-(2,2-dichloroethenyl)-2,2-dimethyl-4-(1*H*-benzimidazol-2-yl)phenyl-cyclopropanecarboxylic acid ester (known from CN 103524422 A) (CAS 1542271-46-4); (4a*S*)-7-chloro-2,5-dihydro-2-[[(methoxycarbonyl) [4-[(trifluoromethyl)thio]phenyl]amino]carbonyl]-indeno[1,2-*e*][1,3,4]oxadiazine-4a(3*H*)-carboxylic acid methyl ester (known from CN 102391261 A) (CAS 1370358-69-2); 6-deoxy-3-*O*-ethyl-2,4-di-*O*-methyl-, 1-[*N*-[4-[1-[4-(1,1,2,2,2-pentafluoroethoxy)phenyl]-1*H*-1,2,4-triazol-3-yl]phenyl]carbamate]-α-L-mannopyranose (known from US 2014/0275503 A1) (CAS 1181213-14-8); 8-(2-cyclopropylmethoxy-4-trifluoromethyl-phenoxy)-3-(6-trifluoromethyl-pyridazin-3-yl)-3-aza-bicyclo[3.2.1]octane (CAS 1253850-56-4), (8-*anti*)-8-(2-cyclopropylmethoxy-4-trifluoromethyl-phenoxy)-3-(6-trifluoromethyl-pyridazin-3-yl)-3-aza-bicyclo[3.2.1]octane (CAS 933798-27-7), (8-*syn*)-8-(2-cyclopropylmethoxy-4-trifluoromethyl-phenoxy)-3-(6-trifluoromethyl-pyridazin-3-yl)-3-aza-bicyclo[3.2.1]octane (known from WO 2007040280 A1, WO 2007040282 A1) (CAS 934001-66-8), N-[3-chloro-1-(3-pyridinyl)-1H-pyrazol-4-yl]-N-ethyl-3-[(3,3,3-trifluoropropyl)thio]-propanamide (known from WO 2015/058021 A1, WO 2015/058028 A1) (CAS 1477919-27-9) and N-[4-(aminothioxomethyl)-2-methyl-6-[(methylamino)carbonyl]phenyl]-3-bromo-1-(3-chloro-2-pyridinyl)-1*H*-pyrazole-5-carboxamide (known from CN 103265527 A) (CAS 1452877-50-7), 5-(1,3-dioxan-2-yl)-4-[[4-(trifluoromethyl)phenyl] methoxy]-pyrimidine (known from WO 2013/115391 A1) (CAS 1449021-97-9), 3-(4-chloro-2,6-dimethylphenyl)-4-hydroxy-8-methoxy-1-methyl-1,8-diazaspiro[4.5]dec-3-en-2-one (known from WO 2010/066780 A1, WO 2011/151146 A1) (CAS 1229023-34-0), 3-(4-chloro-2,6-dimethylphenyl)-8-methoxy-1-methyl-1,8-diazaspiro[4.5]decane-2,4-dione (known from WO 2014/187846 A1) (CAS 1638765-58-8), 3-(4-chloro-2,6-dimethylphenyl)-8-methoxy-1-methyl-2-oxo-1,8-diazaspiro[4.5]dec-3-en-4-yl-carbonic acid ethyl ester (known from WO 2010/066780 A1, WO 2011151146 A1) (CAS 1229023-00-0), N-[1-[(6-chloro-3-pyridinyl)methyl]-2(1*H*)-pyridinylidene]-2,2,2-trifluoro-acetamide (known from DE 3639877 A1, WO 2012029672 A1) (CAS 1363400-41-2), [N(*E*)]-N-[1-[(6-chloro-3-pyridinyl)methyl] -2(1H)-pyridinylidene]-2,2,2-trifluoro-acetamide, (known from WO 2016005276 A1) (CAS 1689566-03-7), [N(Z)]-N-[1-[(6-chloro-3-pyridinyl)methyl]-2(1H)-pyridinylidene]-2,2,2-trifluoro-acetamide, (CAS 1702305-40-5), 3-*endo*-3-[2-propoxy-4-(trifluoromethyl)phenoxy]-9-[[5-(trifluoromethyl)-2-pyridinyl] oxy]-9-azabicyclo[3.3.1]nonane (known from WO 2011/105506 A1, WO 2016/133011 A1) (CAS 1332838-17-1).

Examples of safeners which can be used in the method of the invention are, for example, benoxacor, cloquintocet (-mexyl), cyometrinil, cyprosulfamide, dichlormid, fenchlorazole (-ethyl), fenclorim, flurazole, fluxofenim, furilazole, isoxadifen (-ethyl), mefenpyr (-diethyl), naphthalic anhydride, oxabetrinil, 2-methoxy-N-({4-[(methylcarbamoyl)amino]phenyl}-sulphonyl)benzamide (CAS 129531-12-0), 4-(dichloroacetyl)-1-oxa-4-azaspiro[4.5]decane (CAS 71526-07-3), 2,2,5-trimethyl-3-(dichloroacetyl)-1,3-oxazolidine (CAS 52836-31-4).

Examples of herbicides which can be used in the method of the invention are:

Acetochlor, acifluorfen, acifluorfen-sodium, aclonifen, alachlor, allidochlor, alloxydim, alloxydim-sodium, ametryn, amicarbazone, amidochlor, amidosulfuron, 4-amino-3-chloro-6-(4-chloro-2-fluoro-3-methylphenyl)-5-fluoropyridine-2-carboxylic acid, aminocyclopyrachlor, aminocyclopyrachlor-potassium, aminocyclopyrachlor-methyl, aminopyralid, amitrole, ammoniumsulfamate, anilofos, asulam, atrazine, azafenidin, azimsulfuron, beflubutamid, benazolin, benazolin-ethyl, benfluralin, benfuresate, bensulfuron, bensulfuron-methyl, bensulide, bentazone, benzobicyclon, benzofenap, bicyclopyron, bifenox, bilanafos, bilanafos-sodium, bispyribac, bispyribac-sodium, bromacil, bromobutide, bromofenoxim, bromoxynil, bromoxynil-butyrate, -potassium, -heptanoate, and -octanoate, busoxinone, butachlor, butafenacil, butamifos, butenachlor, butralin, butroxydim, butylate, cafenstrole, carbetamide, carfentrazone, carfentrazone-ethyl, chloramben, chlorbromuron, chlorfenac, chlorfenac-sodium, chlorfenprop, chlorflurenol, chlorflurenol-methyl, chloridazon, chlorimuron, chlorimuron-ethyl, chlorophthalim, chlorotoluron, chlorthal-dimethyl, chlorsulfuron, cinidon, cinidon-ethyl, cinmethylin, cinosulfuron, clacyfos, clethodim, clodinafop, clodinafop-propargyl, clomazone, clomeprop, clopyralid, cloransulam, cloransulam-methyl, cumyluron, cyanamide, cyanazine, cycloate, cyclopyrimorate, cyclosulfamuron, cycloxydim, cyhalofop, cyhalofop-butyl, cyprazine, 2,4-D, 2,4-D-butotyl, -butyl, -dimethylammonium,-diolamin, -ethyl, -2-ethylhexyl, -isobutyl, -isooctyl, -isopropylammonium, -potassium,-triisopropanolammonium, and -trolamine, 2,4-DB, 2,4-DB-butyl, -dimethylammonium, -isooctyl,-potassium, and -sodium, daimuron (dymron), dalapon, dazomet, n-decanol, desmedipham, detosyl-pyrazolate (DTP), dicamba, dichlobenil, 2-(2,4-dichlorobenzyl)-4,4-dimethyl-1,2-oxazolidin-3-one, 2-(2,5-dichlorobenzyl)-4,4-dimethyl-1,2-oxazolidin-3-one, dichlorprop, dichlorprop-P, diclofop, diclofop-methyl, diclofop-P-methyl, diclosulam, difenzoquat, diflufenican, diflufenzopyr, diflufenzopyr-sodium, dimefuron, dimepiperate, dimethachlor, dimethametryn, dimethenamid, dimethenamid-P, dimetrasulfuron, dinitramine, dinoterb, diphenamid, diquat, diquat-dibromid, dithiopyr, diuron, DNOC, endothal, EPTC, esprocarb, ethalfluralin, ethametsulfuron, ethametsulfuron-methyl, ethiozin, ethofumesate, ethoxyfen, ethoxyfen-ethyl, ethoxysulfuron, etobenzanid, F-9600, F-5231, i.e. N-{2-chloro-4-fluoro-5-[4-(3-fluoropropyl)-5-oxo-4,5-dihydro-1H-tetrazol-1-yl]phenyl}ethanesulfonamide, F-7967, i. e. 3-[7-chloro-5-fluoro-2-(trifluoromethyl)-1H-benzimidazol-4-yl]-1-methyl-6-(trifluoromethyl)pyrimidine-2,4(1H,3H)-dione, fenoxaprop, fenoxaprop-P, fenoxaprop-ethyl, fenoxaprop-P-ethyl, fenoxasulfone, fenquinotrione, fentrazamide, flamprop, flamprop-M-isopropyl, flamprop-M-methyl, flazasulfuron, florasulam, fluazifop, fluazifop-P, fluazifop-butyl, fluazifop-P-butyl, flucarbazone, flucarbazone-sodium, flucetosulfuron, fluchloralin, flufenacet, flufenpyr, flufenpyr-ethyl, flumetsulam, flumiclorac, flumiclorac-pentyl, flumioxazin, fluometuron, flurenol, flurenol-butyl, -dimethylammonium and -methyl, fluoroglycofen, fluoroglycofen-ethyl, flupropanate, flupyrsulfuron, flupyrsulfuron-methyl-sodium, fluridone, fluro-chloridone, fluroxypyr, fluroxypyr-meptyl, flurtamone, fluthiacet, fluthiacet-methyl, fomesafen, fomesafen-sodium, foramsulfuron, fosamine, glufosinate, glufosinate-ammonium, glufosinate-P-sodium, glufosinate-P-ammonium, glufosinate-P-sodium, glyphosate, glyphosate-ammonium, -isopropylammonium, -diammonium, -dimethylammonium, -potassium, -sodium, and -trimesium, H-9201, i.e. O-(2,4-dimethyl-6-nitrophenyl) O-ethyl isopropylphosphoramidothioate, halauxifen, halauxifen-methyl, halosafen, halosulfuron, halosulfuron-methyl, haloxyfop, haloxyfop-P, haloxyfop-ethoxyethyl, haloxyfop-P-ethoxyethyl, haloxyfop-methyl, haloxyfop-P-methyl, hexazinone, HW-02, i.e. 1-(dimethoxyphosphoryl) ethyl-(2,4-dichlorophenoxy)acetate, imazamethabenz, imazamethabenz-methyl, imazamox, imazamox-ammonium, imazapic, imazapic-ammonium, imazapyr, imazapyr-isopropylammonium, imazaquin, imazaquin-ammonium, imazethapyr, imazethapyr-immonium, imazosulfuron, indanofan, indaziflam, iodosulfuron, iodosulfuron-methyl-sodium, ioxynil, ioxynil-octanoate, -potassium and -sodium, ipfencarbazone, isoproturon, isouron, isoxaben, isoxaflutole, karbutilate, KUH-043, i.e. 3-({[5-(difluoromethyl)-1-methyl-3-(trifluoromethyl)-1H-pyrazol-4-yl]methyl}sulfonyl)-5,5-dimethyl-4,5-dihydro-1,2-oxazole, ketospiradox, lactofen, lenacil, linuron, MCPA, MCPA-butotyl,-dimethylammonium, -2-ethylhexyl, -isopropylammonium, -potassium, and -sodium, MCPB, MCPB-methyl, -ethy,l and -sodium, mecoprop, mecoprop-sodium, and -butotyl, mecoprop-P, mecoprop-P-butotyl, -dimethylammonium, -2-ethylhexyl, and -potassium, mefenacet, mefluidide, mesosulfuron, mesosulfuron-methyl, mesotrione, methabenzthiazuron, metam, metamifop, metamitron, metazachlor, metazosulfuron, methabenzthiazuron, methiopyrsulfuron, methiozolin, methyl isothiocyanate, metobromuron, metolachlor, S-metolachlor, metosulam, metoxuron, metribuzin, metsulfuron, metsulfuron-methyl, molinat, mono-linuron, monosulfuron, monosulfuron-ester, MT-5950, i.e. N-(3-chloro-4-isopropylphenyl)-2-methylpentan amide, NGGC-011, napropamide, NC-310, i.e. [5-(benzyloxy)-1-methyl-1H-pyrazol-4-yl](2,4-dichlorophenyl)methanone, neburon, nicosulfuron, nonanoic acid (pelargonic acid), norflurazon, oleic acid (fatty acids), orbencarb, orthosulfamuron, oryzalin, oxadiargyl, oxadiazon, oxasulfuron, oxaziclomefon, oxyfluorfen, paraquat, paraquat dichloride, pebulate, pendimethalin, penoxsulam, pentachlorphenol, pentoxazone, pethoxamid, petroleum oils, phenmedipham, picloram, picolinafen, pinoxaden, piperophos, pretilachlor, primisulfuron, primisulfuron-methyl, prodiamine, profoxydim, prometon, prometryn, propachlor, propanil, propaquizafop, propazine, propham, propisochlor, propoxycarbazone, propoxycarbazone-sodium, propyrisulfuron, propyzamide, prosulfocarb, prosulfuron, pyraclonil, pyraflufen, pyraflufen-ethyl, pyrasulfotole, pyrazolynate (pyrazolate), pyrazosulfuron, pyrazosulfuron-ethyl, pyrazoxyfen, pyribambenz, pyribambenz-isopropyl, pyribambenz-propyl, pyribenzoxim, pyributicarb, pyridafol, pyridate, pyriftalid, pyriminobac, pyriminobac-methyl, pyrimi-sulfan, pyrithiobac, pyrithiobac-sodium, pyroxasulfone, pyroxsulam, quinclorac, quinmerac, quinoclamine, quizalofop, quizalofop-ethyl, quizalofop-P, quizalofop-P-ethyl, quizalofop-P-tefuryl, rimsulfuron, saflufenacil, sethoxydim, siduron, simazine, simetryn, SL-261, sulcotrion, sulfentrazone, sulfometuron, sulfometuron-methyl, sulfosulfuron, SYN-523, SYP-249, i.e. 1-ethoxy-3-methyl-1-oxobut-3-en-2-yl 5-[2-chloro-4-(trifluoromethyl)phenoxy]-2-nitrobenzoate, SYP-300, i.e. 1-[7-fluoro-3-oxo-4-(prop-2-yn-1-yl)-3,4-dihydro-2H-1,4-benzoxazin-6-yl]-3-propyl-2-thioxoimidazolidine-4,5-dione, 2,3,6-TBA, TCA (trichloroacetic acid), TCA-sodium, tebuthiuron, tefuryltrione, tembotrione, tepraloxydim, terbacil, terbucarb, terbumeton, terbuthylazin, terbutryn, thenylchlor, thiazopyr, thiencarbazone, thiencarbazone-methyl, thifensulfuron, thifensulfuron-methyl, thiobencarb, tiafenacil, tolpyralate, topramezone, tralkoxydim, triafamone, tri-allate, triasulfuron, triaziflam, tribenuron, tribenuron-methyl, triclopyr, trietazine, trifloxysulfuron, trifloxysulfuron-sodium, trifludimoxazin, trifluralin, triflusulfuron, triflusulfuron-methyl, tritosulfuron, urea sulfate, vernolate, XDE-848, ZJ-0862, i.e. 3,4-dichloro-N-{2-[(4,6-dimethoxypyrimidin-2-yl)oxy]benzyl}aniline, and the following compounds:

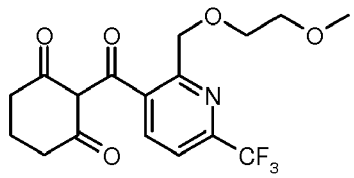
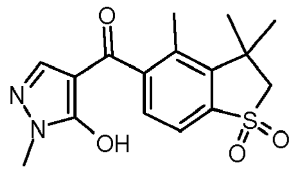
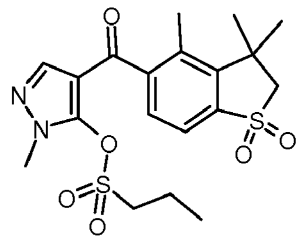

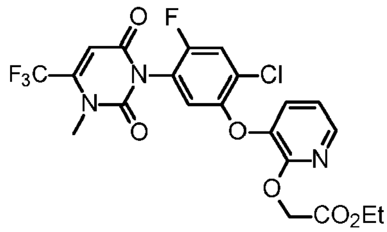

Examples for plant growth regulators are:

Acibenzolar, acibenzolar-S-methyl, 5-aminolevulinic acid, ancymidol, 6-benzylaminopurine, Brassinolid, catechine, chlormequat chloride, cloprop, cyclanilide, 3-(cycloprop-1-enyl) propionic acid, daminozide, dazomet, n-decanol, dikegulac, dikegulac-sodium, endothal, endothal-dipotassium, -disodium, and-mono(N,N-dimethylalkylammonium), ethephon, flumetralin, flurenol, flurenol-butyl, flurprimidol, forchlorfenuron, gibberellic acid, inabenfide, indol-3-acetic acid (IAA), 4-indol-3-ylbutyric acid, isoprothiolane, probenazole, jasmonic acid, maleic hydrazide, mepiquat chloride, 1-methylcyclopropene, methyl jasmonate, 2-(1-naphthyl)acetamide, 1-naphthylacetic acid, 2- naphthyloxyacetic acid, nitrophenolate-mixture, paclobutrazol, N-(2-phenylethyl)-beta-alanine, N-phenylphthalamic acid, prohexadione, prohexadione-calcium, prohydrojasmone, salicylic acid, strigolactone, tecnazene, thidiazuron, triacontanol, trinexapac, trinexapac-ethyl, tsitodef, uniconazole, uniconazole-P.

The invention is illustrated by the examples below. However, the invention is not limited to the examples.

## Examples

### Example 1:

**Efficacy of different compounds of formula (I) and standard azole-type fungicides against leaf diseases in Brazilian maize**

In 2017/2018 five field trials in maize were conducted in order to study the effect of compounds of formula (I) and standard azole-type fungicides regarding disease control against leaf diseases and crop safety.

The trials were done under practical field conditions in Brazil in the Safrinha season with the commercially available maize varieties P30F53 Pioneer, P30F53 RR - Roundup Ready® Pioneer, P30F53YHV Pioneer, DKB 390 Dekalb® and DKB 390 IPRO3 Dekalb®.

These trials were conducted in the following federal states in Brazil: Goias, Mato Grosso, Minas Gerais, Parana and Sao Paulo.

The tested compounds / reference products, their respective formulation type and rates of application [g a.i./ha] (a.i. = active ingredient) are listed in table 1.

Tab.1: Products, formulations and rates.

| Product | Active ingredient | Formulation*3 | Rate (g a.i./ha) |
|---|---|---|---|
| FOX® XPRO * | Bixafen 125 + Prothioconazole 175 + Trifloxystrobin 150 | 450 SC | Bixafen 62.5 + Prothioconazole 87.5 + Trifloxystrobin 75 |
| PROLINE® *1 | Prothioconazole | 250 EC | 125 |
| SCORE® *1 | Difenoconazole | 250 EC | 125 |
| (I-01) *2 | (I-01) | 050 EC | 125 |

(continued)

| Product | Active ingredient | Formulation*3 | Rate (g a.i./ha) |
|---|---|---|---|
| (I-02) *2 | (I-02) | 050 EC | 125 |
| * commercially available product comprising 125 g/l Bixafen, 175 g/l Prothioconazole, 150 g/l Trifloxystrobin and surfactants. This product is regarded to be the present market standard.<br>*1 commercially available products.<br>*2 (1.01) = 2-[6-(4-chlorophenoxy)-2-(trifluoromethyl)-3-pyridyl]-1-(1,2,4-triazol-1-yl)propan-2-ol; (1.02) = 2-[6-(4-bromophenoxy)-2-(trifluoromethyl)-3-pyridyl]-1-(1,2,4-triazol-1-yl)propan-2-ol<br>*3 SC = suspension concentrate; EC = emulsifiable concentrate; the number indicates the amount of a.i. in the formulation [g/l]. | | | |

The products were sprayed twice in sequential, foliar applications at growth stages BBCH EC 39 - 55 and BBCH EC 51 - 69 with a spray interval of about 2 weeks with backpack or bicycle applicators.

The water quantity for the applications was 150 l / ha.

About 14 - 28 days after the second application crop response and disease control were assessed visually by rating the damaged leaves or plant parts.

Crop response (PTX = phytotoxicity), disease control against different pathogens and yield data are shown in table 2.

The efficacy against the pathogens listed in table 2 was calculated according to Abbott's formula:

$$\text{Efficacy } (\%) = (X - Y) / X \times 100,$$

wherein X is the disease severity in the control and Y is the disease severity in the treatment,
and is shown in table 2 [%].

The yield of the untreated control (43 dt/ha) is set to 100% and the yield values for the other treatments are given in percent thereof.

Tab. 2: Impact of foliar applications of tested compounds on crop response, disease control and yield.

| Product | Rate (g a.i. / ha) | PTX (%) 5*6 | Cercospora zeae maydis (% efficacy) 2*6 | Phaeosphaeria maydis (% efficacy) 3*6 | Helminthosporium spp. (% efficacy) 3*6 | Puccinia sorghi (% efficacy) 2*6 | Puccinia polysora (% efficacy) 1*6 | Yield (% of Control) 3*6 |
|---|---|---|---|---|---|---|---|---|
| Control *4 | - | 0 | 0 (40 % disease severity) | 0 (44 % disease severity) | 0 (22 % disease severity) | 0 (20 % disease severity) | 0 (73 % disease severity) | 100 |
| FOX® XPRO * | 225 *5 | 0 | 46 | 52 | 49 | 61 | 77 | 125 |
| PROLINE® *1 | 125 | 0 | 50 | 50 | 42 | 31 | 71 | 109 |
| SCORE® *1 | 125 | 0 | 23 | 41 | 39 | 42 | 80 | 121 |
| (I-01) *2 | 125 | 0 | 61 | 68 | 65 | 71 | 76 | 123 |
| (I-02) *2 | 125 | 0 | 70 | 64 | 63 | 68 | 74 | 112 |
| *, *1, *2: see table 1<br>*4 untreated control, i.e. no application of a fungicide<br>*5 62.5 g/ha Bixafen, 87.5 g/ha Prothioconazole, 75 g/ha Trifloxystrobin<br>*6 number of trials | | | | | | | | |

Based on these results it can be concluded that the tested compounds of formula (I):

- were completely safe on the crop.
- showed a broad disease control spectrum on a set of typical leaf spot and rust diseases.
- showed an overall disease control on a high level and better than the market standard Fox® Xpro.
- had a positive impact on yield.
- delivered a clear superior disease control compared to the straight standard SBI products Score® and Proline® and thus a technical progress is obvious.

**Example 2:**

**Efficacy of different compounds of formula (I) and standard azole-type fungicides against leaf diseases in North American maize**

In 2018 eight field trials in maize were conducted in order to study the effect of compounds of formula (I) and standard azole-type fungicides regarding disease control against leaf diseases.

The trials were done under practical field conditions in Canada and the USA with the commercially available maize varieties DKC 62-08 Dekalb®, DKC 35-88 RIB RR2 BT Dekalb®, P7958 AM RR2 LL BT (Marc Hutlet Seeds Ltd.), NK1120-3122 RR+GLU+ECB+CRW (Syngenta), P9608AM LL RR2 CRW ECB (Pioneer) and Golden Bantam (conventional variety available from various companies worldwide).

These trials were conducted in the following provinces in Canada and federal states in North America: Ontario (Canada) and Georgia, Indiana, Iowa, Michigan, Ohio (all USA).

The tested compounds / reference products, their respective formulation type and rates of application [g a.i./ha] (a.i. = active ingredient) are listed in table 3.

Tab.3: Products, formulations and rates.

| Product | Active ingredient | Formulation*3 | Rate (g a.i./ha) |
|---|---|---|---|
| DELARO®* | Prothioconazole 175 + Trifloxystrobin 150 | 325 SC | Prothioconazole 100-102 + Trifloxystrobin 86-88 |
| PROLINE® *1 | Prothioconazole | 480 SC | 200 |
| (I-02) *2/** | (I-02) | 100 EC or 250 SC ** | 60 -125 |

* commercially available product comprising 175 g/l Prothioconazole, 150 g/l Trifloxystrobin and surfactants. This product is regarded to be the present market standard.
*1 commercially available products.
*2 (1.02) = 2-[6-(4-bromophenoxy)-2-(trifluoromethyl)-3-pyridyl]-1-(1,2,4-triazol-1-yl)propan-2-ol.
** formulation details are summarized in table 4.
*3 SC = suspension concentrate; EC = emulsifiable concentrate; the number indicates the amount of a.i. in the formulation [g/l].

In table 4 details of the formulations of (1-02) used in the different trials are listed.

Tab.4: Formulations of (1-02) and a tank mixed surfactant used in different trials.

| Trial | Country | Federal State or Province | Formulation of (I-02) |
|---|---|---|---|
| 1-3 | CAN | Ontario | SC 250, in tank mixture with surfactant Induce, 900 SL * |
| 4 | USA | Georgia | EC 100 |
| 5 | USA | Indiana | SC 250, in tank mixture with surfactant Induce, 900 SL * |
| 6 | USA | Iowa | SC 250, in tank mixture with surfactant Induce, 900 SL * |

(continued)

| Trial | Country | Federal State or Province | Formulation of (I-02) |
|---|---|---|---|
| 7 | USA | Michigan | EC 100 in tank mixture with surfactant Induce, 900 SL * |
| 8 | USA | Ohio | EC 100 |
| * Induce® = commercial non ionic surfactant | | | |

The products were sprayed with one foliar application at growth stage BBCH EC 63 - 65 with a high clearance sprayer or a backpack applicator.

The water quantity for the applications varied from 150 to 300 l/ha.

About 23 - 35 days after application the disease control was assessed visually by rating the damaged leaves or plant parts.

Crop response (PTX = phytotoxicity), disease control against different pathogens and yield data are shown in table 5.

The efficacy against the pathogens listed in table 5 was calculated according to Abbott's formula:

$$\text{Efficacy (\%)} = (X - Y) / X \times 100,$$

wherein X is the disease severity in the control and Y is the disease severity in the treatment, and is shown in table 5 [%].

The yield of the untreated control (112 dt/ha) is set to 100% and the yield values for the other treatments are given in percent thereof

Tab. 5: Impact of foliar applications of tested compounds on disease control.

| Product | Rate (g a.i./ ha) | PTX (%) 3*6 | Cochliobolus heterostrophus (% efficacy) 1*6 | Kabatiella zeae (% efficacy) 1*6 | Setosphaeria turcica (% efficacy) 2*6 | Yield (% of Control) 2*6 |
|---|---|---|---|---|---|---|
| Control *4 | - | 0 | 0 (12 % disease severity) | 0 (17 % disease severity) | 0 (18 % disease severity) | 100 |
| DELARO * | 188 *5 | 0 | 50 | 65 | 36 | 111 |
| PROLINE®*1 | 200 | 0 | 34 | 41 | 40 | 107 |
| (I-02) *2 | 60 | 0 | 91 | 89 | 57 | 120 |
| (I-02) *2 | 125 | 0 | 97 | 95 | 61 | 115 |
| *, *1, *2: see table 3<br>*4 untreated control, i.e. no application of a fungicide<br>*5 100-102 g/ha Prothioconazole, 86-88 g/ha Trifloxystrobin<br>*6 number of trials | | | | | | |

We can sum up the above mentioned data:

- Compound (1-02) showed a broad disease control spectrum on a set of typical leaf spot diseases.
- This overall disease control was on a high level and better than the market standard Delaro®.
- Compound (1-02) was completely safe on the crop and did not generate any symptoms of phytotoxicity.
- Compound (1-02) had a positive impact on yield and increased the level of grain harvest.

- Compared to the straight standard SBI product Proline® there also is a clear superior disease control and increased harvest level delivered by (1-02) and thus a technical progress is obvious.

## Claims

**1.** Method of controlling fungi causing rust or leaf disease in maize plants, comprising spray application of a compound of formula (I)

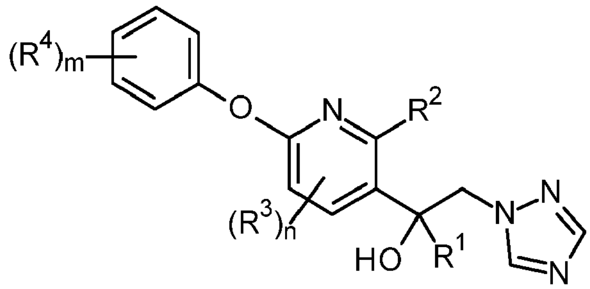

(I),

wherein

$R^1$ represents hydrogen, $C_1$-$C_6$-alkyl or $C_3$-$C_8$-cycloalkyl,
wherein the $C_1$-$C_6$-alkyl may carry 1, 2, 3 or up to the maximum possible number of identical or different groups $R^a$ which independently of one another are selected from halogen, CN, nitro, phenyl, $C_1$-$C_4$-alkoxy and $C_1$-$C_4$-halogenalkoxy,
and wherein the $C_3$-$C_8$-cycloalkyl may carry 1, 2, 3, 4, 5 or up to the maximum number of identical or different groups $R^b$ which independently of one another are selected from halogen, CN, nitro, $C_1$-$C_4$-alkyl, $C_1$-$C_4$-alkoxy, $C_1$-$C_4$-halogenalkyl and $C_1$-$C_4$-halogenalkoxy;
$R^2$ represents $C_1$-$C_2$-halogenalkyl or halogen;
each $R^3$ represents independently from each other halogen, CN, nitro, $C_1$-$C_4$-alkyl, $C_1$-$C_4$-halogenalkyl, $C_1$-$C_4$-alkoxy or $C_1$-$C_4$-halogenalkoxy;
n is an integer and is 0 or 1;
each $R^4$ represents independently of one another halogen, CN, nitro, $C_1$-$C_4$-alkyl, $C_1$-$C_4$-halogenalkyl, $C_1$-$C_4$-alkoxy, $C_1$-$C_4$-halogenalkoxy, $C_1$-$C_4$-alkylcarbonyl, hydroxy-substituted $C_1$-$C_4$-alkyl, pentafluoro-$\lambda^6$-sulfanyl, $C_3$-$C_6$-cycloalkyl, $C_3$-$C_6$-halogencycloalkyl or $C_1$-$C_4$-alkyl-$C_3$-$C_6$-cycloalkyl; and
m is an integer and is 0, 1 or 2;

to the maize plant or a part thereof.

**2.** Method according to claim 1, wherein

$R^1$ represents hydrogen, methyl, $CF_3$, ethyl, n-propyl, isopropyl, n-butyl, *tert.*-butyl, cyclopropyl, 1-fluorocyclopropyl, 1-chlorocyclopropyl or 1-methylcyclopropyl,
each $R^4$ represents independently from each other $CF_3$, $OCF_3$, Br, Cl, or pentafluoro-$\lambda^6$-sulfanyl,
and
m is 1.

**3.** Method according to at least one of claims 1 to 2, wherein

$R^2$ represents $CF_3$ or Cl,
and
n is 0.

**4.** Method according to at least one of claims 1 to 3, wherein the compound of formula (I) is selected from the group consisting of 2-[6-(4-chlorophenoxy)-2-(trifluoromethyl)-3-pyridyl]-1-(1,2,4-triazol-1-yl)propan-2-ol and 2-[6-(4-bromophenoxy)-2-(trifluoromethyl)-3-pyridyl]-1-(1,2,4-triazol-1-yl)propan-2-ol.

5. Method according to at least one of claims 1 to 4, wherein the compound of formula (I) is selected from the group consisting of (2R)-2-[6-(4-chlorophenoxy)-2-(trifluoromethyl)-3-pyridyl]-1-(1,2,4-triazol-1-yl)propan-2-ol, (2S)-2-[6-(4-chlorophenoxy)-2-(trifluoromethyl)-3-pyridyl]-1-(1,2,4-triazol-1-yl)propan-2-ol, the racemate of (2R)-2-[6-(4-chlorophenoxy)-2-(trifluoromethyl)-3-pyridyl]-1-(1,2,4-triazol-1-yl)propan-2-ol and (2S)-2- [6-(4-chlorophenoxy)-2-(trifluoromethyl)-3-pyridyl]-1-(1,2,4-triazol-1-yl)propan-2-ol, any mixture of (2R)-2-[6-(4-chlorophenoxy)-2-(trifluoromethyl)-3-pyridyl]-1-(1,2,4-triazol-1-yl)propan-2-ol and (2S)-2- [6-(4-chlorophenoxy)-2-(trifluoromethyl)-3-pyridyl]-1-(1,2,4-triazol-1-yl)propan-2-ol, (2R)-2- [6-(4-bromophenoxy)-2-(trifluoromethyl)-3-pyridyl]-1-(1,2,4-triazol-1-yl)propan-2-ol, (2S)-2- [6-(4-bromophenoxy)-2-(trifluoromethyl)-3-pyridyl]-1-(1,2,4-triazol-1-yl)propan-2-ol, the racemate of (2R)-2-[6-(4-bromophenoxy)-2-(trifluoromethyl)-3-pyridyl]-1-(1,2,4-triazol-1-yl)propan-2-ol and (2S)-2-[6-(4-bromophenoxy)-2-(trifluoromethyl)-3-pyridyl]-1-(1,2,4-triazol-1-yl)propan-2-ol, and any mixture of (2R)-2-[6-(4-bromophenoxy)-2-(trifluoromethyl)-3-pyridyl]-1-(1,2,4-triazol-1-yl)propan-2-ol and (2S)-2-[6-(4-bromophenoxy)-2-(trifluoromethyl)-3-pyridyl]-1-(1,2,4-triazol-1-yl)propan-2-ol.

6. Method according to at least one of claims 1 to 5, wherein the leaf disease is selected from leaf blotch, leaf wilt, leaf spot and blight diseases.

7. Method according to at least one of claims 1 to 6, wherein the fungus is selected from *Cercospora* species, *Phaeosphaeria* species, *Helminthosporium* species, *Puccinia* species, *Cochliobolus* species, *Kabatiella* species and *Setosphaeria* species.

8. Method according to claim 7, wherein the fungus is selected from *Cercospora zeae maydis, Phaeosphaeria maydis, Helminthosporium spp., Puccinia sorghi, Puccinia polysora, Cochliobolus heterostrophus, Kabatiella zeae* and *Setosphaeria turcica.*

9. Method according to at least one of claims 1 to 8, wherein the compound of formula (I) is applied to the maize plant or apart thereof 1 to 10 times.

10. Method according to at least one of claims 1 to 9, wherein the compound of formula (I) is applied to the maize plant or a part thereof at least once in growth stages BBCH 30 to 70.

11. Method according to at least one of claims 1 to 10, wherein the compound of formula (I) is provided as emulsifiable concentrate comprising 20 to 400 g/l of the compound of formula (I) or as suspension concentrate comprising 100 to 500 g/l of the compound of formula (I).

12. Method according to claim 11, wherein the emulsifiable concentrate or suspension concentrate is mixed with water in such amount to arrive at a concentration of the compound of formula (I) in the resulting mixture of 0.1 to 5 g/l.

13. Method according to at least one of claims 1 to 12, wherein the compound of formula (I) is applied to the maize plant or part thereof in an amount corresponding to 50 to 150 g compound of formula (I) per hectare treated area.

14. Method according to at least one of claims 1 to 13, wherein the maize is selected from the Pioneer maize varieties P30F53, P30F53 RR - Roundup Ready®, P9608AM LL RR2 CRW ECB and P30F53YHV, the Dekalb® maize varieties DKB 390, DKB 390 IPRO3, DKC 35-88 RIB RR2 BT and DKC 62-08, the variety P7958 AM RR2 LL BT available from Marc Hutlet Seeds Ltd., the variety NK1120-3122 RR+GLU+ECB+CRW available from Syngenta and the variety Golden Bantam.

Europäisches Patentamt
European Patent Office
Office européen des brevets

# EUROPEAN SEARCH REPORT

Application Number
EP 18 21 1902

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | EP 3 421 460 A1 (BAYER AG [DE]) 2 January 2019 (2019-01-02) * the whole document * ----- | 1-14 | INV. A01N43/653 C07D401/06 A01P3/00 |
| X,D | WO 2017/029179 A1 (BAYER CROPSCIENCE AG [DE]) 23 February 2017 (2017-02-23) | 1-14 | |
| Y | * the claims; the examples; page 34; page 35, lines 19-38; pages 36-38; page 39, lines 4-38; pages 40-43; page 44; page 48, lines 21-37; page 49, lines 1-18; page 56, lines 9-29 * ----- | 1-14 | |
| X | WO 2018/145934 A1 (BAYER CROPSCIENCE AG [DE]; BAYER AG [DE]) 16 August 2018 (2018-08-16) | 1,3,6-9, 13 | |
| Y | * the claims; the examples; page 40, lines 5-34; page 41; page 42, lines 1-2; page 47; page 48, lines 1-9; page 49, lines 10-31; page 50, lines 12-36; pages 51-56; page 57, last paragraph; page 62, lines 12-31, and page 64, lines 6-27 * ----- | 1-14 | TECHNICAL FIELDS SEARCHED (IPC) |
| X | WO 2018/145933 A1 (BAYER AG [DE]; BAYER CROPSCIENCE AG [DE]) 16 August 2018 (2018-08-16) | 1-14 | A01N C07D |
| Y | * the claims; the examples; page 26, lines 7-17; page 28, lines 20-35; page 29, lines 1-31; page 30, lines 1-22; page 36, lines 7-35; page 37, second paragraph; page 39, lines 5-37; page 40, lines 6-34; pages 41-45; page 46, lines 20-26; page 50, lines 23-33; page 52, lines 27-33; page 53 * ----- | 1-14 | |

-/--

The present search report has been drawn up for all claims

1

| Place of search | Date of completion of the search | Examiner |
|---|---|---|
| Munich | 16 January 2019 | Lorenzo Varela, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 18 21 1902

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | WO 2018/145921 A1 (BAYER AG [DE]; BAYER CROPSCIENCE AG [DE])<br>16 August 2018 (2018-08-16)<br>* the claims; the examples; page 36, lines 9-32; pages 37-38; page 40, lines 20-35; pages 41-42; page 43, lines 1-24; page 46, third paragraph; page 48, lines 3-32; page 52, lines 23-33; page 53, first paragraph; page 54, lines 31-35; page 55 *<br>----- | 1-9,13<br>1-14 | |
| X<br>Y | EP 3 391 747 A1 (BAYER AG [DE])<br>24 October 2018 (2018-10-24)<br>* the claims; paragraphs 1-8, 28-34, 42-49 and the examples *<br>----- | 1-14<br>1-14 | |

TECHNICAL FIELDS SEARCHED (IPC)

The present search report has been drawn up for all claims

1

| Place of search | Date of completion of the search | Examiner |
|---|---|---|
| Munich | 16 January 2019 | Lorenzo Varela, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 18 21 1902

This annex lists the patent family members relating to the patent documents cited in the above-mentioned European search report.
The members are as contained in the European Patent Office EDP file on
The European Patent Office is in no way liable for these particulars which are merely given for the purpose of information.

16-01-2019

| Patent document cited in search report | Publication date | Patent family member(s) | Publication date |
|---|---|---|---|
| EP 3421460 A1 | 02-01-2019 | NONE | |
| WO 2017029179 A1 | 23-02-2017 | AU 2016310123 A1 | 01-03-2018 |
| | | CA 2995304 A1 | 23-02-2017 |
| | | CN 108137538 A | 08-06-2018 |
| | | CO 2018001456 A2 | 30-04-2018 |
| | | CR 20180102 A | 11-05-2018 |
| | | EA 201890490 A1 | 31-08-2018 |
| | | EP 3334718 A1 | 20-06-2018 |
| | | JP 2018526359 A | 13-09-2018 |
| | | KR 20180037267 A | 11-04-2018 |
| | | PE 6032018 A1 | 09-04-2018 |
| | | TW 201718500 A | 01-06-2017 |
| | | US 2018235223 A1 | 23-08-2018 |
| | | UY 36852 A | 31-03-2017 |
| | | WO 2017029179 A1 | 23-02-2017 |
| WO 2018145934 A1 | 16-08-2018 | NONE | |
| WO 2018145933 A1 | 16-08-2018 | NONE | |
| WO 2018145921 A1 | 16-08-2018 | UY 37605 A | 28-09-2018 |
| | | WO 2018145921 A1 | 16-08-2018 |
| EP 3391747 A1 | 24-10-2018 | NONE | |



For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2017029179 A1
- WO 2018145921 A1
- US 6060051 A
- US 7094592 B
- WO 2016154297 A
- US 6245551 B
- TW 4764
- TW 5454
- TW 5096
- TW 5277
- EP 718408 A
- EP 701275 A
- IT 2008000196 W
- WO 2006043635 A
- WO 2003106457 A
- WO 2006003494 A
- WO 2010052161 A
- EP 2647626 A
- WO 2004099160 A
- JP 2010018586 A
- WO 2012029672 A
- WO 2013144213 A
- WO 2010051926 A
- CN 103232431
- WO 2013050317 A1
- WO 2013162715 A2
- WO 2013162716 A2
- US 20140213448 A1
- CN 101337937 A
- CN 103109816 A
- WO 2012034403 A1
- WO 2011085575 A1
- CN 101337940 A
- CN 101715774 A
- CN 103524422 A
- CN 102391261 A
- US 20140275503 A1
- WO 2007040280 A1
- WO 2007040282 A1
- WO 2015058021 A1
- WO 2015058028 A1
- CN 103265527 A
- WO 2013115391 A1
- WO 2010066780 A1
- WO 2011151146 A1
- WO 2014187846 A1
- DE 3639877 A1
- WO 2012029672 A1
- WO 2016005276 A1
- WO 2011105506 A1
- WO 2016133011 A1

### Non-patent literature cited in the description

- Growth stages of mono- and dicotyledonous plants. Federal Biological Research Centre for Agriculture and Forestry, 2001
- **ONGENA, M. et al.** Bacillus Lipopeptides: Versatile Weapons for Plant Disease Biocontrol. *Trends in Microbiology,* March 2008, vol. 16 (3), 115-125
- *CHEMICAL ABSTRACTS,* 885026-50-6
- *CHEMICAL ABSTRACTS,* 637360-23-7
- *CHEMICAL ABSTRACTS,* 872999-66-1
- *CHEMICAL ABSTRACTS,* 1225292-17-0
- *CHEMICAL ABSTRACTS,* 1440516-42-6
- *CHEMICAL ABSTRACTS,* 792914-58-0
- *CHEMICAL ABSTRACTS,* 1204776-60-2
- *CHEMICAL ABSTRACTS,* 1363400-41-2
- *CHEMICAL ABSTRACTS,* 1461743-15-6
- *CHEMICAL ABSTRACTS,* 1226889-14-0
- *CHEMICAL ABSTRACTS,* 1449220-44-3
- *CHEMICAL ABSTRACTS,* 1332628-83-7
- *CHEMICAL ABSTRACTS,* 1477923-37-7
- *CHEMICAL ABSTRACTS,* 1105672-77-2
- *CHEMICAL ABSTRACTS,* 1232543-85-9
- *CHEMICAL ABSTRACTS,* 1268277-22-0
- *CHEMICAL ABSTRACTS,* 1233882-22-8
- *CHEMICAL ABSTRACTS,* 1108184-52-6
- *CHEMICAL ABSTRACTS,* 1542271-46-4
- *CHEMICAL ABSTRACTS,* 1370358-69-2
- *CHEMICAL ABSTRACTS,* 1181213-14-8
- *CHEMICAL ABSTRACTS,* 1253850-56-4
- *CHEMICAL ABSTRACTS,* 933798-27-7
- *CHEMICAL ABSTRACTS,* 934001-66-8
- *CHEMICAL ABSTRACTS,* 1477919-27-9
- *CHEMICAL ABSTRACTS,* 1452877-50-7
- *CHEMICAL ABSTRACTS,* 1449021-97-9
- *CHEMICAL ABSTRACTS,* 1229023-34-0
- *CHEMICAL ABSTRACTS,* 1638765-58-8
- *CHEMICAL ABSTRACTS,* 1229023-00-0
- *CHEMICAL ABSTRACTS,* 1689566-03-7

- *CHEMICAL ABSTRACTS,* 1702305-40-5
- *CHEMICAL ABSTRACTS,* 1332838-17-1
- *CHEMICAL ABSTRACTS,* 129531-12-0
- *CHEMICAL ABSTRACTS,* 71526-07-3
- *CHEMICAL ABSTRACTS,* 52836-31-4